# EUROPEAN PATENT APPLICATION

(11) **EP 3 722 295 A1**
(43) Date of publication of application: **14.10.2020**
(21) Application number: 19305479.8
(22) Date of filing: 12.04.2019
(51) Int. Cl.: C07D 413/14, A61K 31/4245, A61K 31/519, C07D 487/04, A61P 35/00, A61P 31/12

(54) **ANTI-VIRAL AND ANTI-CANCER ACTIVITY OF PYRIDO[2,3-D]PYRIMIDINE AND OXADIAZIOLE COMPOUNDS**

(71) Applicant: CENTRE NATIONAL DE LA RECHERCHE SCIENTIFIQUE, 75016 Paris (FR); Institut Pasteur, 75724 Paris Cedex 15 (FR); UNIVERSITE PARIS DESCARTES, 75006 Paris (FR); Université Grenoble Alpes, 38400 Saint-Martin-d'Hères (FR)
(72) Inventor: NAFFAKH, Nadia, 92240 Malakoff (FR); CREPIN, Thibaut, 38100 Grenoble (FR); VIDALAIN, Pierre-Olivier, 69006 LYON (FR); PIETRANCOSTA, Nicolas, 78180 MONTIGNY LE BRETONNEUX (FR); LE CORRE, Laurent, 92340 BOURG-LA-REINE (FR); BUSCA, Patricia, 92120 MONTROUGE (FR); ASHRAF, Usama, 92200 Neuilly-sur-Seine (FR); CORIO, Alessandra, 75012 PARIS (FR); GRAVIER-PELLETIER, Christine, 91540 Mennecy (FR)
(74) Representative: Lavoix

(57) **Abstract**

The present invention relates to compounds of formula (A), their enantiomers and their pharmaceutically acceptable salts, as RED-SMU1 disruptors. These compounds may be useful in the treatment and/or prevention of virus infections, cancer, but also for in vitro applications. In Formula (A), U, V and W are each independently an atom chosen from C, N, O and S; R₁ is a radical chosen from C₁-C₆-alkoxy, -NO₂, -NH₂, -COOH, halogen and -O-CH₂-CH₂-COOH; n is an integer comprised between 1 and 3; R₂ may be present or absent, and when present, is a radical chosen from -NH-CO-NH-Alk, -NH-CS-NH-Alk and -CH₂-CO-NH-Alk, wherein Alk is a C₁-C₆ alkyl; Het is a heteroaryl which may be fused to the aromatic cycle comprising U, V and W, and which may be substituted; R₃ is a radical chosen from -phenyl-triazole-aralkyl, -NH-(CH₂)m-alkyne, -NH-(CH₂)m-OH, -NH-(CH₂)m-triazole-fluorophore and -NH-(CH₂)m-triazole-heterocycle; m is an integer comprised between 1 and 5; t is equal to 0 or 1; and R₄ is a radical chosen from H, halogen and -C(R₅)(R₆)-CH₂OH, wherein R₅ and R₆ each represent H or a C₁-C₆ alkyl.

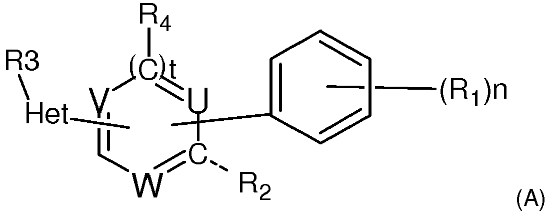

SEQUENCE LISTING

<110>  CNRS et al

<120>  Anti-viral activity of pyrido[2,3-d]pyrimidine compounds

<130>  BET 19P1157

<160>  5

<170>  PatentIn version 3.5

<210>  1
<211>  100
<212>  PRT
<213>  Homo sapiens

<400>  1

Leu Leu Gln Lys Val Arg Ala Glu Ile Ala Ser Lys Glu Lys Glu Glu
1               5                   10                  15

Glu Glu Leu Met Glu Lys Pro Gln Lys Glu Thr Lys Lys Asp Glu Asp
            20                  25                  30

Pro Glu Asn Lys Ile Glu Phe Lys Thr Arg Leu Gly Arg Asn Val Tyr
        35                  40                  45

Arg Met Leu Phe Lys Ser Lys Ala Tyr Glu Arg Asn Glu Leu Phe Leu
    50                  55                  60

Pro Gly Arg Met Ala Tyr Val Val Asp Leu Asp Asp Glu Tyr Ala Asp
65                  70                  75                  80

Thr Asp Ile Pro Thr Thr Leu Ile Arg Ser Lys Ala Asp Cys Pro Thr
                85                  90                  95

Met Glu Ala Gln
            100

<210>  2
<211>  111
<212>  PRT
<213>  Caenorhabditis elegans

<400>  2

Leu Leu Asn Lys Val Arg Ser Glu Ile Asp Lys Ser Asp Asp Asp Asp
1               5                   10                  15

Asp Asp Asp Ile Asp Thr Ala Phe Asp Glu Lys Val Thr Ser Ser Ser
            20                  25                  30

Ser Ser Ser Lys Pro Ser Glu Ala Ser Leu Leu Ala Gln Glu Leu Ala

35                  40                  45

Gln Ser His Ser Glu Asn Arg Met Val Arg Ser Leu His Arg Val Leu
    50                  55                  60

Phe Lys Asn Glu Val Pro Leu His Asn Gln Leu Phe Ala Lys Gly Arg
65                  70                  75                  80

Met Ala Tyr Val Val Glu Leu Glu Asp Glu Glu Thr Asp Ile Pro Thr
                85                  90                  95

Thr Leu Leu Arg Ser Leu His Asp Leu Pro Arg Ala Glu Ser Ala
            100                 105                 110

<210>  3
<211>  196
<212>  PRT
<213>  Homo sapiens

<400>  3

Met Ser Ile Glu Ile Glu Ser Ser Asp Val Ile Arg Leu Ile Met Gln
1               5                   10                  15

Tyr Leu Lys Glu Asn Ser Leu His Arg Ala Leu Ala Thr Leu Gln Glu
            20                  25                  30

Glu Thr Thr Val Ser Leu Asn Thr Val Asp Ser Ile Glu Ser Phe Val
        35                  40                  45

Ala Asp Ile Asn Ser Gly His Trp Asp Thr Val Leu Gln Ala Ile Gln
    50                  55                  60

Ser Leu Lys Leu Pro Asp Lys Thr Leu Ile Asp Leu Tyr Glu Gln Val
65                  70                  75                  80

Val Leu Glu Leu Ile Glu Leu Arg Glu Leu Gly Ala Ala Arg Ser Leu
                85                  90                  95

Leu Arg Gln Thr Asp Pro Met Ile Met Leu Lys Gln Thr Gln Pro Glu
            100                 105                 110

Arg Tyr Ile His Leu Glu Asn Leu Leu Ala Arg Ser Tyr Phe Asp Pro
        115                 120                 125

Arg Glu Ala Tyr Pro Asp Gly Ser Ser Lys Glu Lys Arg Arg Ala Ala
    130                 135                 140

Ile Ala Gln Ala Leu Ala Gly Glu Val Ser Val Val Pro Pro Ser Arg

145                 150                 155                 160

Leu Met Ala Leu Leu Gly Gln Ala Leu Lys Trp Gln Gln His Gln Gly
                165                 170                 175

Leu Leu Pro Pro Gly Met Thr Ile Asp Leu Phe Arg Gly Lys Ala Ala
            180                 185                 190

Val Lys Asp Val
        195

<210>  4
<211>  196
<212>  PRT
<213>  Caenorhabditis elegans

<400>  4

Met Ser Ser Ile Glu Ile Glu Ser Ser Asp Val Ile Arg Leu Ile Glu
1               5                   10                  15

Gln Phe Leu Lys Glu Ser Asn Leu His Arg Thr Leu Ala Ile Leu Gln
            20                  25                  30

Glu Glu Thr Asn Val Ser Leu Asn Thr Val Asp Ser Ile Asp Gly Phe
        35                  40                  45

Cys Asn Glu Ile Thr Ser Gly Asn Trp Asp Asn Val Leu Lys Thr Val
    50                  55                  60

Gln Ser Leu Lys Leu Pro Ala Lys Lys Leu Ile Asp Leu Tyr Glu His
65                  70                  75                  80

Val Ile Ile Glu Leu Val Glu Leu Arg Glu Leu Ala Thr Ala Arg Leu
                85                  90                  95

Val Ala Arg Gln Thr Asp Pro Met Ile Leu Leu Lys Gln Ile Asp Pro
            100                 105                 110

Asp Arg Phe Ala Arg Leu Glu Ser Leu Ile Asn Arg Pro Tyr Phe Asp
        115                 120                 125

Gly Gln Glu Val Tyr Gly Asp Val Ser Lys Glu Lys Arg Arg Ser Val
    130                 135                 140

Ile Ala Gln Thr Leu Ser Ser Glu Val His Val Val Ala Pro Ser Arg
145                 150                 155                 160

Leu Leu Ser Leu Leu Gly Gln Ser Leu Lys Trp Gln Leu His Gln Gly

165                 170                 175

Leu Leu Pro Pro Gly Thr Ala Ile Asp Leu Phe Arg Gly Lys Ala Ala
            180                 185                 190

Gln Lys Glu Gln
        195

<210>  5
<211>  202
<212>  PRT
<213>  Homo sapiens

<400>  5

Met Ser Ile Glu Ile Glu Ser Ser Asp Val Ile Arg Leu Ile Met Gln
1               5                   10                  15

Tyr Leu Lys Glu Asn Ser Leu His Arg Ala Leu Ala Thr Leu Gln Glu
            20                  25                  30

Glu Thr Thr Val Ser Leu Asn Thr Val Asp Ser Ile Glu Ser Phe Val
        35                  40                  45

Ala Asp Ile Asn Ser Gly His Trp Asp Thr Val Leu Gln Ala Ile Gln
    50                  55                  60

Ser Leu Lys Leu Pro Asp Lys Thr Leu Ile Asp Leu Tyr Glu Gln Val
65                  70                  75                  80

Val Leu Glu Leu Ile Glu Leu Arg Glu Leu Gly Ala Ala Arg Ser Leu
                85                  90                  95

Leu Arg Gln Thr Asp Pro Met Ile Met Leu Lys Gln Thr Gln Pro Glu
            100                 105                 110

Arg Tyr Ile His Leu Glu Asn Leu Leu Ala Arg Ser Tyr Phe Asp Pro
        115                 120                 125

Arg Glu Ala Tyr Pro Asp Gly Ser Ser Lys Glu Lys Arg Arg Ala Ala
    130                 135                 140

Ile Ala Gln Ala Leu Ala Gly Glu Val Ser Val Val Pro Pro Ser Arg
145                 150                 155                 160

Leu Met Ala Leu Leu Gly Gln Ala Leu Lys Trp Gln Gln His Gln Gly
                165                 170                 175

Leu Leu Pro Pro Gly Met Thr Ile Asp Leu Phe Arg Gly Lys Ala Ala

            180                 185                 190

Val Lys Asp Val Glu Glu Glu Lys Phe Pro
        195                 200

## Description

The present invention concerns the use of specific compounds as RED-SMU1 disruptors and/or destabilizators. Such compounds may be useful for treating and/or preventing viral infections, cancer, but also for *in vitro* applications.

Virus infections, and particularly influenza virus infection, is a serious threat to global public health and there is a critical need for innovative anti-viral drugs. Two broad, non-exclusive approaches to inhibit viral replication and develop novel therapeutic strategies are possible: either targeting directly viral proteins, or targeting host proteins essential for the viral life cycle (host-directed therapies), which is more likely to counter the problem of drug-resistant virus emergence.
In recent years, the concept of host-directed therapies, which target host determinants essential for the infectious life cycle and/or pathogenesis rather than pathogens components, has been rapidly expanding (1, 2). Preclinical studies suggest they could show clinical safety while providing the advantage of broad spectrum efficacy and reduced antiviral resistance. A range of host-directed therapies for several bacterial and viral diseases, with different mechanisms of action, are currently in clinical trials (1, 2).

Influenza A viruses (IAVs) are a leading cause of morbidity and mortality worldwide, as they are responsible for recurring annual epidemics, frequent epizootics, and occasional pandemics (3). The drugs currently licensed for treatment of influenza target viral components (the M2 ion channel, the neuraminidase and the polymerase) all led to the emergence of resistance variants. The M2 inhibitors are no longer recommended for use since currently circulating human H3N2 and H1N1 pdm09 viruses have become naturally resistant to these drugs (4). Although the frequency of resistance to neuraminidase inhibitors (NAIs) of currently circulating human IAVs remains low (around 0.5%) (5), large clusters of H1N1pmd09 viruses resistant to oseltamivir, the most widely used NAI, have been observed (6, 7). The former seasonal H1N1 IAVs had become globally oseltamivir-resistant during the 2007-2008 season (8). Favipiravir, a purine nucleoside analog also known as T-705, is undergoing phase III trials in America and Europe and has been approved in Japan to treat pandemic influenza virus infections. In October 2018, FDA approved Xofluza, a selective inhibitor of the polymerase PA subunit, for the treatment of acute uncomplicated influenza. However, the first evidence for viral adaptation to favipiravir treatment in cell culture has been reported recently (9, 10), and the emergence of PA variants with a mutation conferring resistance to Xofluza was observed in 9.7% of treated patients in the Phase III trial (11).
In this context, novel anti-influenza drugs are actively being sought to efficiently fight IAVs, particularly with regard to pandemic preparedness (12, 13).

Infectious IAV particles contain eight ribonucleoprotein complexes (vRNPs), corresponding to a set of eight distinct viral genomic RNA (vRNA) segments encapsidated with nucleoproteins (NP) and associated with the heterotrimeric viral RNA-dependent RNA polymerase (FluPol) consisting of the PB1, PB2 and PA subunits (3). The viral genome has a limited size (about 13.5 kb), but IAVs have evolved a variety of strategies to expand their coding capacity. Viral mRNAs are synthesized by the FluPol in the nucleus of infected cells. Although most of these are intronless, the M, NS and PB2 segments produce both unspliced mRNAs (M1, NS1, PB2) and spliced mRNAs (M2, NS2, PB2-S1). FluPol recruits a complex formed by the human splicing factors RED (78.9 kDa) and SMU1 (57.5 kDa) by direct binding to RED (23). The complex RED-SMU1 regulates the splicing of viral NS1 mRNA into the mRNA encoding the multifunctional and essential NS2/NEP protein. In cells depleted for RED or SMU1, the production of infectious influenza virions showed a 100-fold reduction (23).

Therefore, there is a need for the development of novel and efficient anti-viral drugs, especially anti-influenza drugs, to counter the problem of viral resistance, to treat critical cases, and/or to bridge the period before a new vaccine becomes available.

The present invention proposes a host-directed therapy which aims to solve these needs: it relates to compounds which target the RED-SMU1 complex and destabilize it.
Indeed, innovatively, the inventors have identified the RED-SMU1 complex as a novel target for viral therapy, and developed a rational screening approach in order to identify compounds that are able to disrupt the RED-SMU1 complex. They first performed subdomain analysis of human RED and SMU1 proteins using cell-based interaction assays, and solved the crystal structure of a minimal RED-SMU1 complex. Then they identified compounds that target an α-helix-groove interface essential for RED-SMU1 interaction. Lastly, they demonstrate the potential of such RED-SMU1 destabilizing compounds for the inhibition of viral infections such as IAV infections.
The identified compounds thus constitute efficient host-directed therapies, particularly for inhibiting viral infections.

Consequently, the present invention first relates to the use of a compound chosen from compounds of formula (A), their enantiomers and their pharmaceutically acceptable salts: 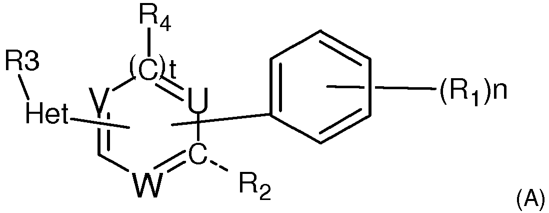 wherein:
U, V and W are each independently an atom chosen from C, N, O and S,
R1 is a radical chosen from C1-C6-alkoxy, -NO2, -NH2, -COOH, halogen and -O-CH2-CH2-COOH,
n is an integer comprised between 1 and 3,
R2 may be present or absent, and when present, is a radical chosen from -NH-CO-NH-Alk, -NH-CS-NH-Alk and -CH2-CO-NH-Alk, wherein Alk is a C1-C6 alkyl,
Het is a heteroaryle which may be fused to the aromatic cycle comprising U, V and W, and which may be substituted,
R3 is a radical chosen from -phenyl-triazole-aralkyle, -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
t is equal to 0 or 1, and R4 is a radical chosen from H, halogen and -C(R5)(R6)-CH2OH, wherein R5 and R6 each represent H or a C1-C6 alkyl,
for preventing and/or treating a viral infection.

By "pharmaceutically acceptable salts", it is meant any salt prepared using an inorganic acid such as hydrochloric acid, sulfuric acid, phosphoric acid or the like, or an organic acid such as acetic acid, citric acid, maleic acid, succinic acid, tartaric acid, lactic acid, malic acid, oxalic acid, fumaric acid, methanesulfonic acid or the like.

Preferably, according to a first embodiment:
U=O,
V = W = N,
t = 0, and
R2 is absent.
The corresponding compounds are of formula (A'): 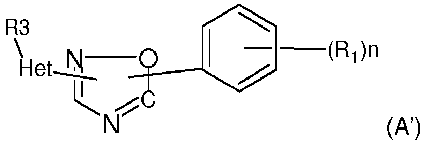 with R1, R3, Het and n as defined above.
More preferably, the compounds of formula (A') are such that the phenyl cycle is linked on the oxadiazole ring on its 5 position.
Preferably, Het is a heteroaryle which is not fused to the aromatic cycle comprising U, V and W, preferably the oxadiazole, and which may be substituted.
By "heteroaryle", it is meant an aryl group in which at least one carbon atom of the aromatic ring is substituted by a heteroatom, and which may be substituted. An aryl is a monocyclic or polycyclic aromatic hydrocarbon group, such as a phenyl group. The heteroatom may be nitrogen, oxygen, phosphorus or sulfur, preferably nitrogen. Examples of heteroaryle include pyrazole, pyrimidine, pyrrole, thiophene, furane, oxazole, and isoxazole groups. Preferably, the heteroaryle is a pyrazole or a pyrimidine. Preferably, the pyrazole is substituted by at least one group -NH-CO-Alk, wherein Alk is a C1-C6 alkyl, and/or by at least one group -COO-Alk, wherein Alk is a C1-C6 alkyl.
Preferably, R3 is -phenyl-triazole-aralkyle.
By "aralkyl", it is meant an aryl group as described above, linked to the -phenyl-triazole-radical by an alkyl group. Preferably, the aralkyl group is benzyl.
Preferably, the compound of formula (A') is compound LSP958 as follows:

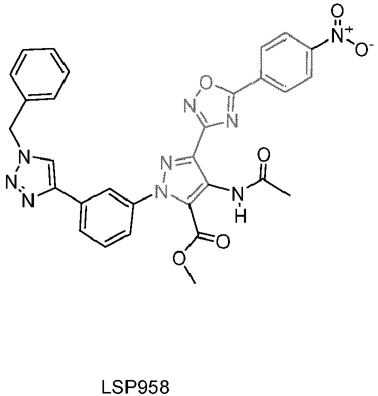

Preferably, according to a second embodiment:
U = V = C,
W = N,
t = 1,
R2 is present,
Het is a heteroaryle which is fused to the aromatic cycle comprising U, V and W, and which is substituted by a further radical R4, and
R3 and R4 are as described above.

Preferably in such a case, the compound of formula (A) is a compound of formula (I) below.

Thus, preferably, the present invention relates to the use of a compound chosen from compounds of formula (I), their enantiomers and their pharmaceutically acceptable salts: 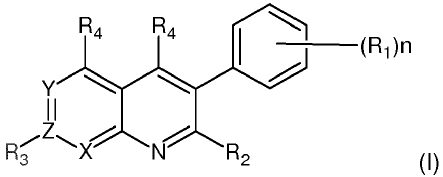 wherein:
X, Y and Z are each independently an atom chosen from C, N, O and S,
R1 is a radical chosen from C1-C6-alkoxy, -NH2, -COOH, halogen and -O-CH2-CH2-COOH,
n is an integer comprised between 1 and 3,
R2 is a radical chosen from -NH-CO-NH-Alk, -NH-CS-NH-Alk and -CH2-CO-NH-Alk, wherein Alk is a C1-C6 alkyl,
R3 is a radical chosen from -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
R4 is a radical chosen from H, halogen and -C(R5)(R6)-CH2OH, wherein R5 and R6 each represent H or a C1-C6 alkyl,
for preventing and/or treating a viral infection.

Preferably, Y and X are each N.
Preferably, Z is C.
n is an integer which may be 1, 2 or 3, preferably 2.

The halogen atom is preferably chosen from F, Br, I and Cl.

By "C1-C6 alkyl", it is meant a linear hydrocarbon group comprising from 1 to 6 carbon atoms, or a branched hydrocarbon group comprising from 3 to 6 carbon atoms. Examples of C1-C6 alkyl groups include methyl, ethyl, n-propyl, isopropyl, n-butyl, isobutyl, tert-butyl, n-pentyl, isopentyl and n-hexyl groups, and preferably methyl, n-butyl, n-pentyl, n-hexyl, isopropyl or tert-butyl. More preferably, the C1-C6 alkyl is methyl or tert-butyl.

By "C1-C6 alkoxy", it is meant an -O-alkyl group wherein the alkyl moiety is a C1-C6 alkyl. Preferably the C1-C6 alkoxy is methoxy.

By "alkyne", it is meant -CΞCH.

By "triazole", it is meant the following radical:

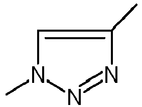

By "fluorophore", it is meant a fluorescent chemical entity, which can re-emit light upon light excitation. A fluorophore typically comprises at least two combined aromatic groups. For example, suitable fluorophores may include a fluorophore selected from a group consisting of fluorescein-type fluorophores, rhodamine-type fluorophores, xanthine-type fluorophores, naphthalene-type fluorophores such as dansyl-type fluorophores, carbocyanine-type fluorophores, coumarin-type fluorophores, acridine-type fluorophores, pyrene-type fluorophores and anthracene-type fluorophores. Preferably, the fluorophore is a fluorescein-type fluorophore, preferably fluorescein.

By "heterocycle", it is meant a monocyclic or polycyclic saturated hydrocarbon group in which at least one carbon atom of the ring is substituted by a heteroatom, and which may be optionally substituted. The heteroatom may be nitrogen, oxygen, or sulfur. Preferably, the heterocycle is morpholino, tetrahydropyrane, oxetane or azetidine, such as 4-tetrahydropyrano or 3-oxetano or 3-azetidino. The heterocycle may be substituted by at least one alkyl group and/or by at least a hydroxyl and/or by at least an aralkyl group such as a benzyl group. Preferably the heterocycle is 1-benzyl-4-morpholino.

Preferably, the compound of formula (I) is of formula (I'): 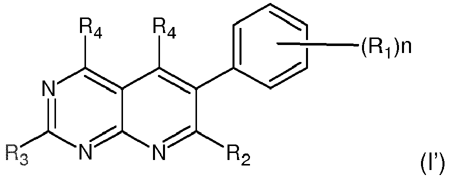 wherein R1, R2, R3, R4, n and m are as described for formula (I).

Preferably, R1 is a C1-C6 alkoxy, more preferably methoxy.
R1 may be in ortho, para or meta position. Preferably, n is 2, and both R1 are in ortho positions.

Preferably, R2 is the radical -NH-CO-NH-Alk, wherein Alk is a C1-C6 alkyl.

Preferably, R3 is a radical chosen from -NH-(CH2)m-alkyne, -NH-(CH2)m-triazole-fluorophore and -NH-(CH2)m-triazole-heterocycle. Preferably, m is 1, 2 or 3.

Preferably, R4 is H.

Preferably, the compound of formula (I) is chosen from compounds LSP641 and LSP61, of structures below:

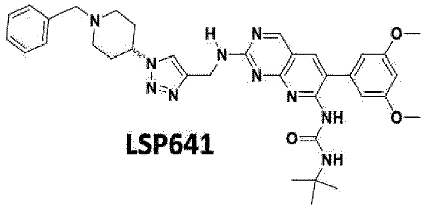

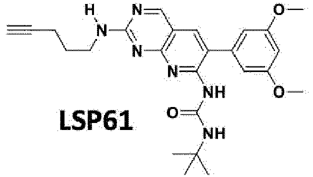

More preferably, the compound is LSP61.

As shown in the examples, the compounds of formula (A) (including formulas (A'), (I) and (I')) of the invention destabilize or disrupt the cellular RED-SMU1 complex.

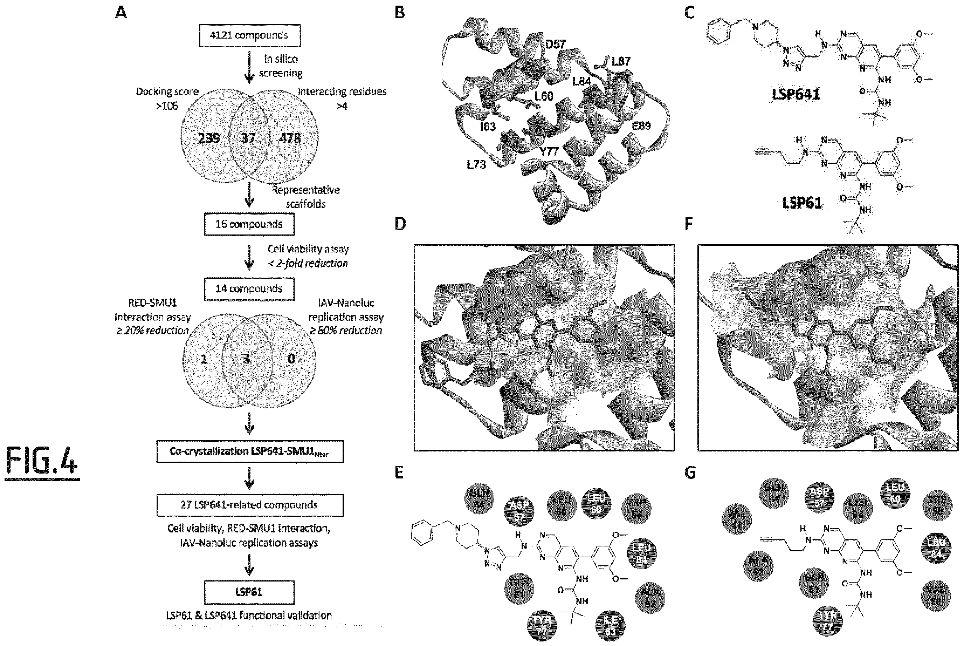

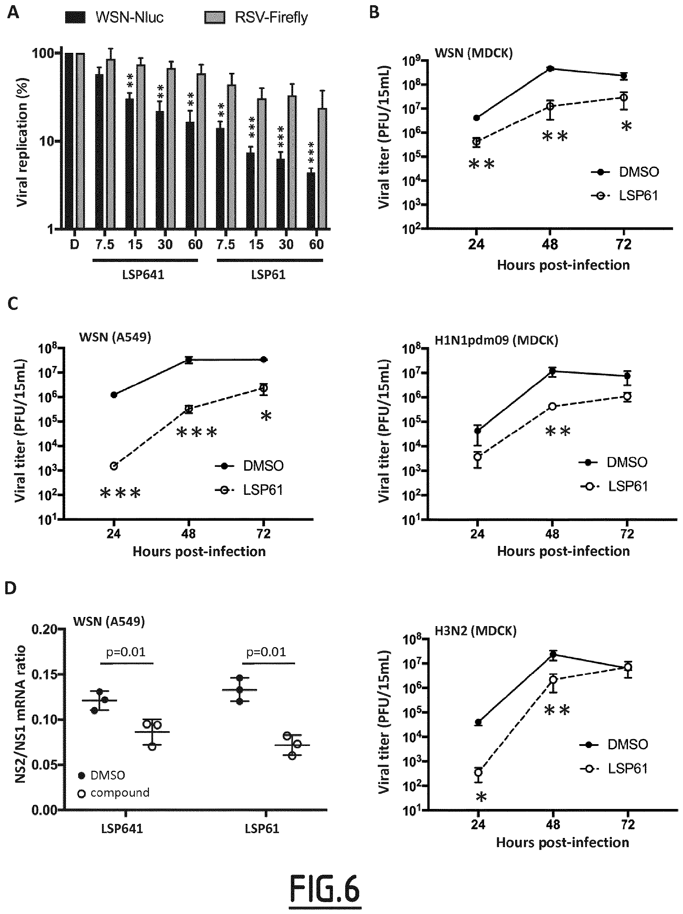

The RED and SMU1 factors are proteins involved in pre-mRNA splicing as components of the spliceosome. The sequence of human RED factor may be found in Uniprot under ID Q13123.
The human SMU1 factor may be found in Uniprot under ID A0MNN4.
The RED-SMU1 complex is part of the host splicing machinery.
As indicated above, in the case of an influenza virus, both factors are required for normal splicing of viral NS1 mRNA into the mRNA encoding the multifunctional and essential NS2 protein, and for the production of viral particles.
By "destabilizing" or "disrupting" the cellular RED-SMU1 complex, it is meant that the compounds of formula (A) of the invention are able to reduce at least 20% of RED-SMU1 interaction. This is shown in Figure 4A. Said reduction of RED-SMU1 interaction may be measured as described the test described in example 1, section "In cellulo split luciferase-based interaction assays". Thus, the compounds of formula (A) according to the invention are able to reduce at least 20% of Gaussia luciferase signal as measured in the test described in example 1, sections "In cellulo split luciferase-based interaction assays" and "Plasmids for split-luciferase-based interaction assays", in the presence of the compound as compared to DMSO.
Preferably, the compounds of formula (A) of the invention further reduce viral replication, such as influenza A virus replication, by at least 80%. This is shown in Figure 4A. The reduction of influenza A virus replication may be measured as described in the test described in example 1, section "Viruses". Thus, the compounds of formula (A) according to the invention are able to reduce at least 80% of Nanoluc signal as measured in the test described in example 1, section "Viruses", in the presence of the compound compared to
DMSO.
Preferably, the compounds of formula (A) of the invention reduce the ratio of NS2/NS1 mRNAs, i.e. reduce the efficiency of the splicing of the viral NS1 mRNA, but do not act on the ratios of M2/M1 mRNAs and of PB2-S1/PB2 mRNAs. This is notably exemplified in the examples and in Figure 6D (for NS2/NS1 mRNA ratio) and Supplementary Figure S6 (for M2/M1 and PB2-S1/PB2 mRNA ratios).

### Antiviral use

According to a first embodiment, the compounds of formula (A) (including formulas (A'), (I) and (I')), their enantiomers and/or their pharmaceutically acceptable salts may be used for preventing and/or treating a viral infection.
By "preventing", it is meant avoiding the viral infection to occur.

By "treatment" is meant the curative treatment of viral infection. A curative treatment is defined as a treatment that completely treat (cure) or partially treat a viral infection.
The "subject" refers to any subject and typically designates a patient afflicted by a viral infection.

By "viral infection", it is meant an infection of a subject by a virus. Said virus is typically a virus which hijacks the host splicing machinery, and thus benefits from the function of the RED-SMU1 complex. The virus may be any virus which replicates in the nucleus of infected cells and produces intron-containing viral mRNAs. In such a case the compound of the present invention typically has a direct mode of action, because it alters the viral mRNA splicing.
Alternatively, typically, the compound of the present invention has an indirect mode of action, and is able to alter a cellular mRNA splicing, which encodes for a protein which plays a role in the viral replication cycle, and the virus may be any type of virus.
The virus may be chosen from influenza viruses, human immunodeficiency viruses (HIV) and human papillomaviruses (HPV).
Influenza virus may be chosen from Influenza virus A, Influenza virus B, Influenza virus C and Influenza virus D.
HIV may be chosen from HIV-1 and HIV-2, preferably is HIV-1. It has to be noted that RED and SMU1 were identified as hits in two high-throughput screens looking for cellular factors involved in HIV-1 life cycle.
HPV may be notably chosen from HPV16, HPV18, HPV6 and HPV11, preferably is HPV18. Indeed, the SMU1 factor was found to be associated to the E6 protein of HPV18.

Preferably, the viral infection is an influenza virus infection, more preferably an Influenza virus A infection.

### Anticancer use

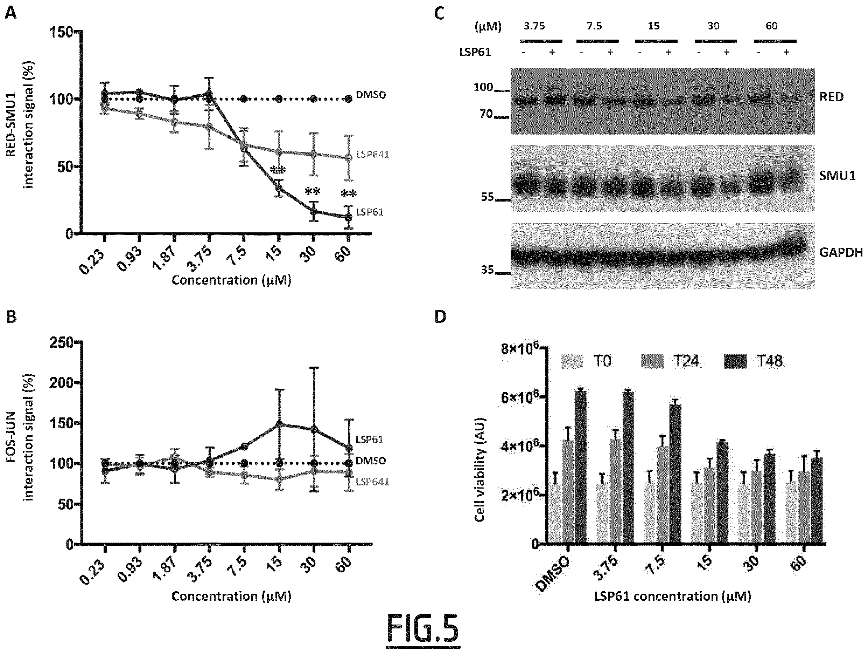

According to a second embodiment, the compounds of formula (A) (including formulas (A'), (I) and (I')), their enantiomers and/or their pharmaceutically acceptable salts may be used for preventing and/or treating cancer.
Indeed, as shown in the examples, these compounds are highly selective for the RED-SMU1 complex. Moreover, the compound LSP61 inhibits the growth of the tumoral cell line A549 (see Figures 5D and S5A).
Moreover, the RED-SMU1 complex regulates the splicing of cellular mRNAs which are involved in cell division control (26).

By "treatment" is meant the curative treatment of cancer. A curative treatment is defined as a treatment that completely treat (cure) or partially treat cancer (i.e. induces tumor growth stabilization, retardation or regression).
The "subject" refers to any subject and typically designates a patient afflicted by cancer.

By "cancer", it is meant any type of cancer. The cancer is for example selected from a colon cancer, a colorectal cancer, a melanoma, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a renal cancer, a skin cancer, a stomach cancer, a testis cancer, an urothelial cancer or an adrenocortical carcinoma, but also non solid cancers such as lymphoma.
The cancer can be a metastatic cancer or not.

In any case, the subject is preferably a vertebrate, more preferably a mammal, even more preferably a human being.

The compound of formula (A) (including formulas (A'), (I) and (I')) of the invention is preferably administered at a therapeutically effective amount or dose. As used herein, "a therapeutically effective amount or dose" refers to an amount of the compound of the invention which prevents, removes, slows down the disease, or reduces or delays one or several symptoms or disorders caused by or associated with said disease in the subject, preferably a human being. The effective amount, and more generally the dosage regimen, of the compound of the invention and pharmaceutical compositions thereof may be determined and adapted by the one skilled in the art. An effective dose can be determined by the use of conventional techniques and by observing results obtained under analogous circumstances. The therapeutically effective dose of the compound of the invention will vary depending on the disease to be treated or prevented, its gravity, the route of administration, any co-therapy involved, the patient's age, weight, general medical condition, medical history, etc.
Typically, the amount of the compound to be administered to a patient may range from about 0.01 to 500 mg/kg of body weight for a human patient. In a particular embodiment, the pharmaceutical composition according to the invention comprises 0.01 mg/kg to 300 mg/kg of the compound of the invention, preferably from 0.01 mg/kg to 3 mg/kg, for instance from 25 to 300 mg/kg.

In a particular aspect, the compounds of the invention can be administered to the subject by parenteral route, topical route, oral route or intravenous injection. The compound or the nanoparticle of the invention may be administered to the subject daily (for example 1, 2, 3, 4, 5, 6 or 7 times a day) during several consecutive days, for example during 2 to 10 consecutive days, preferably from 3 to 6 consecutive days. Said treatment may be repeated during 1, 2, 3, 4, 5, 6 or 7 weeks, or every two or three weeks or every one, two or three months. Alternatively, several treatment cycles can be performed, optionally with a break period between two treatment cycles, for instance of 1, 2, 3, 4 or 5 weeks. The compound of the invention can for example be administered as a single dose once a week, once every two weeks, or once a month. The treatment may be repeated one or several times per year.
Doses are administered at appropriate intervals which can be determined by the skilled person. The amount chosen will depend on multiple factors, including the route of administration, duration of administration, time of administration, the elimination rate of the compound, or of the various products used in combination with said compound, the age, weight and physical condition of the patient and his/her medical history, and any other information known in medicine.

The administration route can be oral, topical or parenteral, typically rectal, sublingual, intranasal, intra-peritoneal (IP), intra-venous (IV), intra-arterial (IA), intra-muscular (IM), intra-cerebellar, intrathecal, intratumoral and/or intradermal. The pharmaceutical composition is adapted for one or several of the above-mentioned routes. The pharmaceutical composition is preferably administered by injection or by intravenous infusion of suitable sterile solutions, or in the form of liquid or solid doses via the alimentary canal.

The compound of formula (A) (including formulas (A'), (I) and (I')), its enantiomers or its pharmaceutically acceptable salts, may be present in a composition comprising a pharmaceutically acceptable carrier.
The carrier must be "acceptable" in the sense of being compatible with the other ingredients of the formulations and not deleterious to the recipient thereof.
The pharmaceutical composition can be formulated as solutions in pharmaceutically compatible solvents or as gels, oils, emulsions, suspensions, or dispersions in suitable pharmaceutical solvents or vehicles, or as pills, tablets, capsules, powders, suppositories, etc. that contain solid vehicles in a way known in the art, possibly through dosage forms or devices providing sustained and/or delayed release. For this type of formulation, an agent such as cellulose, lipids, carbonates or starches are used advantageously.
Agents or vehicles that can be used in the formulations (liquid and/or injectable and/or solid) are excipients or inert vehicles, i.e. pharmaceutically inactive and non-toxic vehicles. Mention may be made, for example, of saline, physiological, isotonic and/or buffered solutions, compatible with pharmaceutical use and known to those skilled in the art. The compositions may contain one or more agents or vehicles chosen from dispersants, solubilizers, stabilizers, preservatives, etc.
Particular examples are methylcellulose, hydroxymethylcellulose, carboxymethylcellulose, cyclodextrins, polysorbate 80, mannitol, gelatin, lactose, liposomes, vegetable oils or animal, acacia, etc. Preferably, vegetable oils are used.
Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.
Formulations suitable for parenteral administration conveniently comprise a sterile oily or aqueous preparation of the active ingredient which is preferably isotonic with the blood of the recipient. Every such formulation can also contain other pharmaceutically compatible and non-toxic auxiliary agents, such as, e.g. stabilizers, antioxidants, binders, dyes, emulsifiers or flavoring substances.

### Novel compounds

The present invention also relates to a compound chosen from compounds of formula (II), their enantiomers and their pharmaceutically acceptable salts: 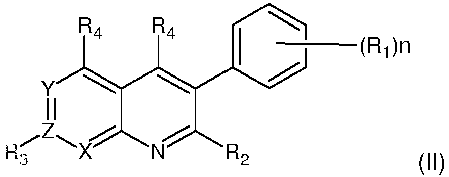 wherein:
X, Y and Z are each independently an atom chosen from C, N, O and S,
R1 is a radical chosen from C1-C6-alkoxy, -NH2, -COOH, halogen and -O-CH2-CH2-COOH,
n is an integer comprised between 1 and 3,
R2 is a radical chosen from -NH-CO-NH-Alk, -NH-CS-NH-Alk and -CH2-CO-NH-Alk, wherein Alk is a C1-C6 alkyl,
R3 is a radical chosen from -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
R4 is a radical chosen from H, halogen and -C(R5)(R6)-CH2OH, wherein R5 and R6 each represent H or a C1-C6 alkyl, provided that either at least one R4 is different from H, or, when both R4 are H, then R3 is -NH-(CH2)m-triazole-heterocycle with the heterocycle being 1-benzyl-4-morpholino.

Said compounds of formula (II) are a subgroup of formula (I), and are useful in therapy. Thus, the invention also relates to the use of at least one compound of formula (II), its enantiomers or its pharmaceutically acceptable salts, as a medicament.
The present invention also relates to a composition comprising, in a pharmaceutically acceptable carrier, at least one compound of formula (II), its enantiomers or its pharmaceutically acceptable salts.

Preferably, the compound of formula (II) is LSP641:

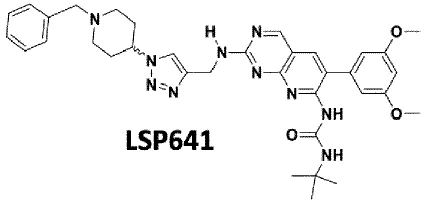

### Preparation of the novel compounds

The compounds of formula (II) according to the present invention may be prepared by a process which is easy to perform.

Such a process may be one similar to the process disclosed in Le Corre et al (32).

### In vitro applications of the compounds of the invention

The compounds of formula (including formulas (A'), (I) and (I')) of the invention may also be useful as *in vitro* tools, notably for high-throughput pharmacological screening (HTS). In the present case, the aim of high-throughput pharmacological screening is to identify compounds that are able to disrupt RED-SMU1 at low concentrations, and are specific for the RED-SMU1 complex i.e. do not show any significant anti-FGFR activity. The HTS may rely on competitive assays.

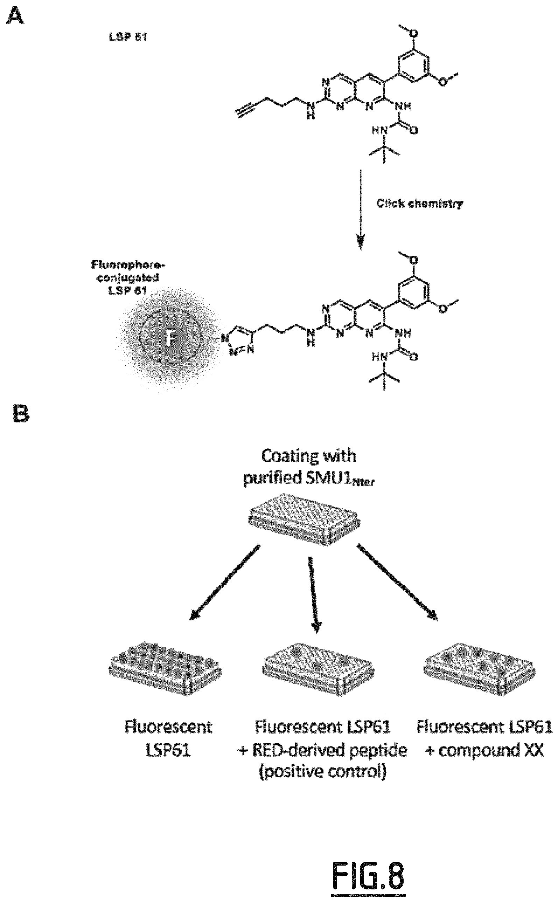

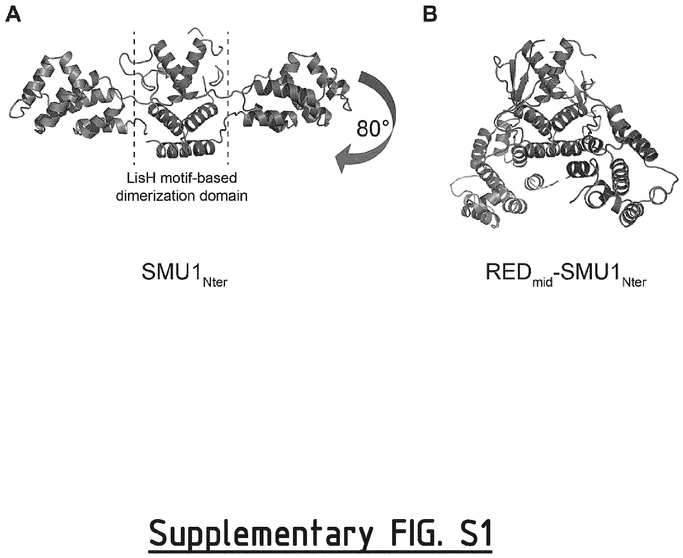

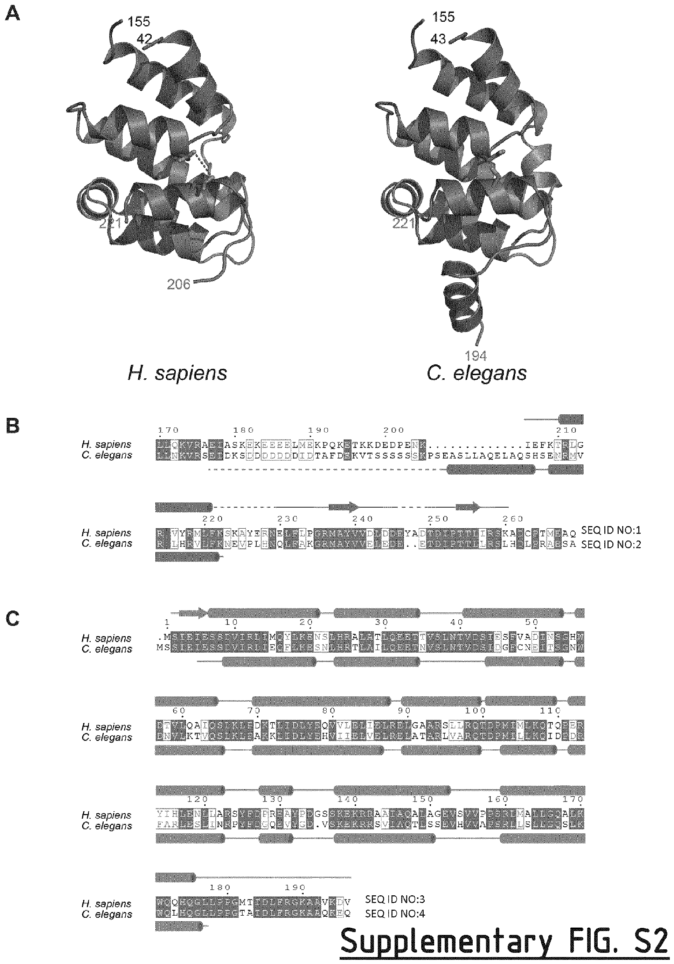

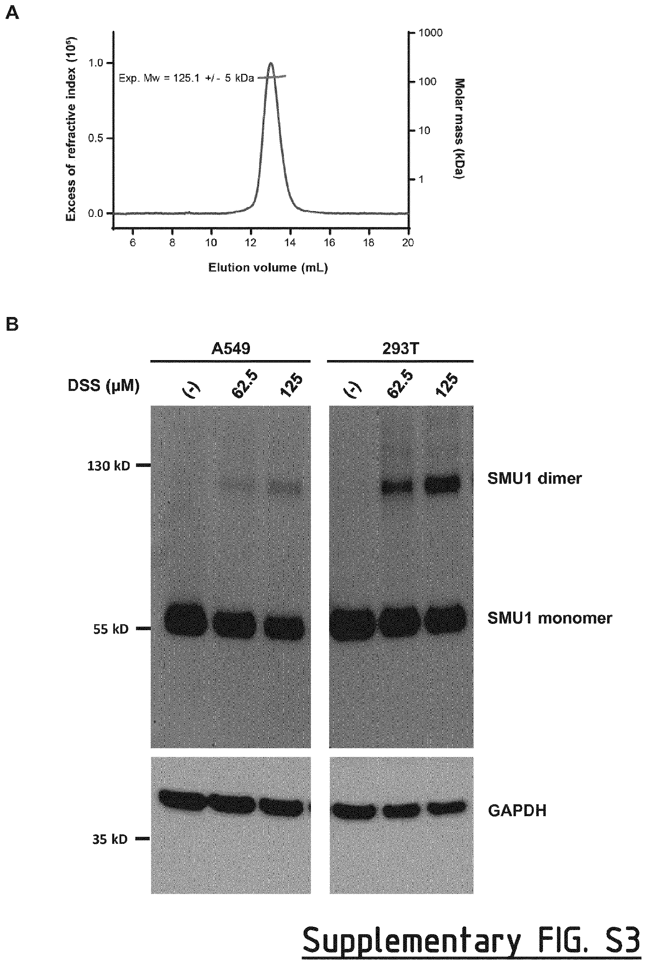

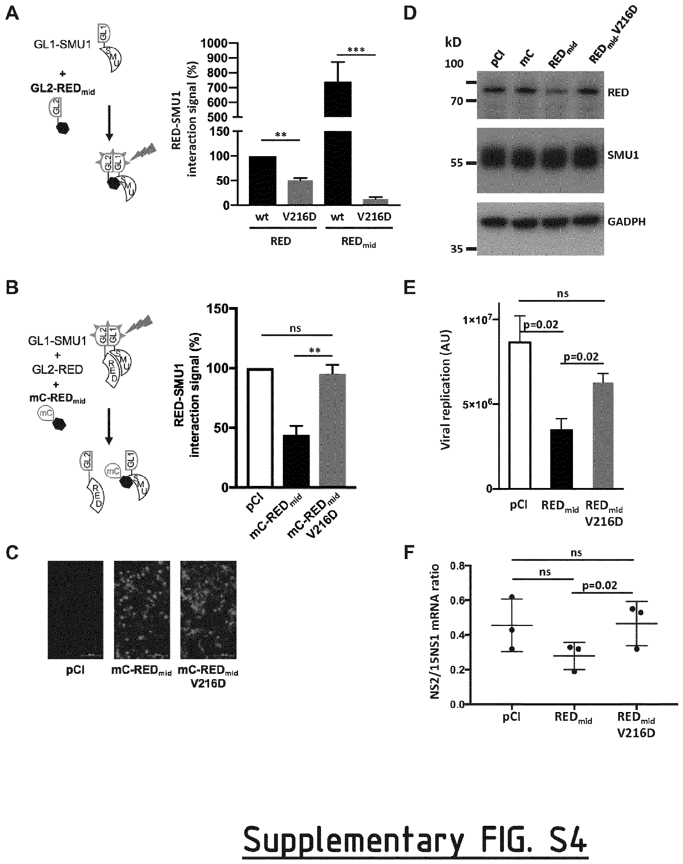

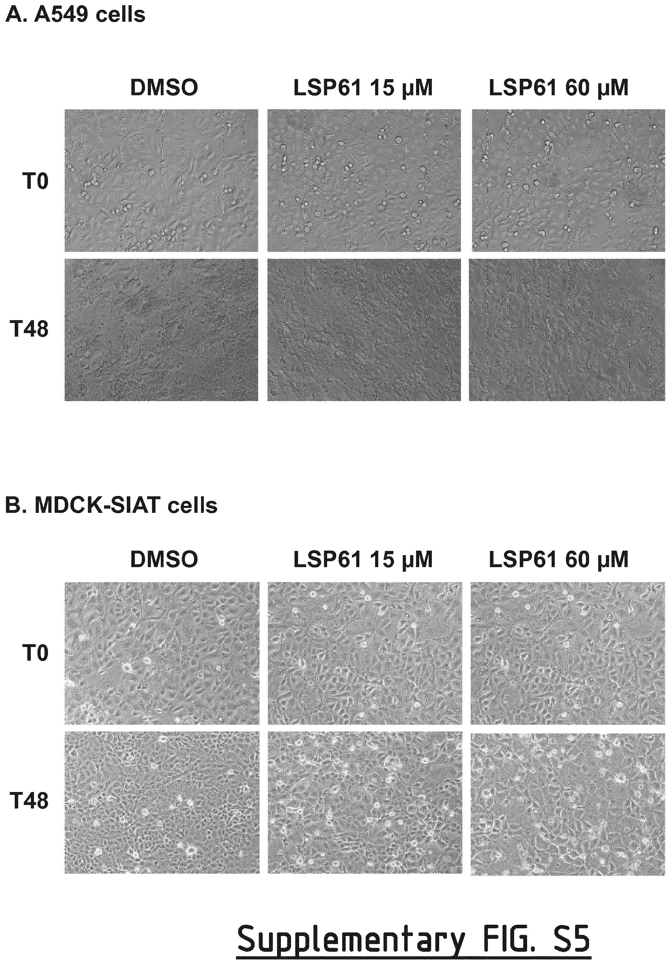

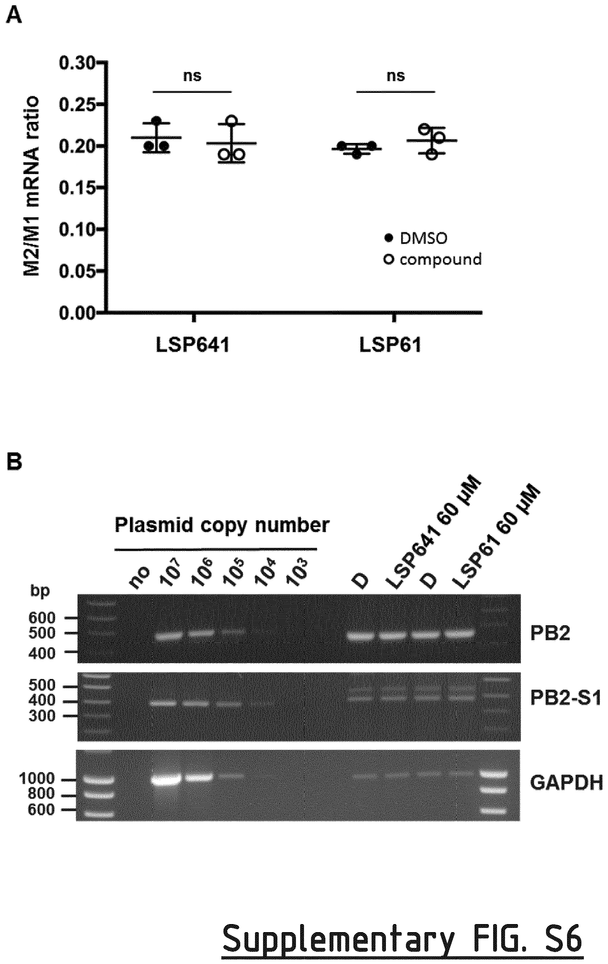

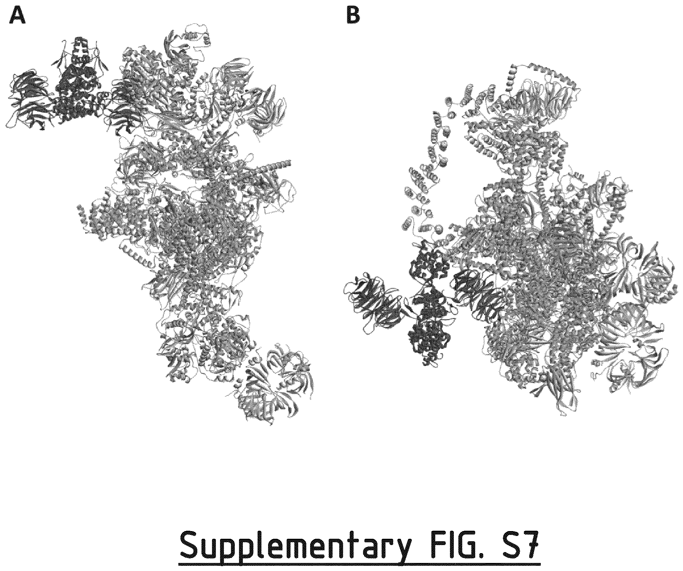

The principle is illustrated in Figure 8.
As shown in figure 8A, the compounds of the invention may be conjugated to a fluorophore by click chemistry, in order to obtain the conjugated derivative. The fluorophore may be as described above.
Then, the obtained conjugated derivative may be used for high-throughput pharmacological screening, in order to identify compounds that bind SMU1_{Nter} (of SEQ ID NO:3 or 5) with a higher affinity and higher specificity than the original compound. For example, when the compound is LSP61, the free alkyne group of LSP61 is conjugated to a fluorophore, and the obtained conjugated LSP61 is then used for high-throughput pharmacological screening, in order to identify compounds that bind SMU1_{Nter} with a higher affinity and higher specificity than LSP61.
The sequence of SMU1_{Nter} which may be used may be SEQ ID NO:3 (which corresponds to residues 1 to 196 of Uniprot ID A0MNN4), or SEQ ID NO:5 (which corresponds to residues 1 to 202 of Uniprot ID A0MNN4, i.e. 
MSIEIESSDVIRLIMQYLKENSLHRALATLQEETTVSLNTVDSIESFVADINSGHWDTVLQAI
QSLKLPDKTLIDLYEQVVLELIELRELGAARSLLRQTDPMIMLKQTQPERYIHLENLLARSY
FDPREAYPDGSSKEKRRAAIAQALAGEVSVVPPSRLMALLGQALKWQQHQGLLPPGMTI
DLFRGKAAVKDVEEEKFP).
 As shown in figure 8B, wells may be coated with SMU1_{Nter} (i.e. solid phase).
Another example of the test may be in liquid phase, i.e. which would be based on an energy transfer (FRET) between the conjugated LSP61 and SMU1_{Nter} fused to a fluorophore such as GFP.
As shown in figure 8B, in a first experiment, the conjugated LSP61 is added and binding is measured.
In a second experiment (positive control), a RED peptide (or RED) and the conjugated LSP61 are added, and the binding is measured. In this second experiment, the RED peptide (or RED) is used in order to inhibit the interaction between the conjugated LSP61 and SMU1_{Nter} The RED peptide is preferably a peptide comprising at least the residues 210 to 222 of the sequence Uniprot ID Q13123, more preferably consists in the residues 210 to 222 of the sequence Uniprot ID Q13123.
In a third experiment, a test compound (compound XX in figure 8B) and the conjugated LSP61 are added, and the binding is measured.

If the binding of the conjugated LSP61 in the third experiment is less than the binding of the conjugated LSP61 of the first experiment, then the test compound is of pharmacological interest for the present invention, as it competes with LSP61 binding to SMU1, and could thus disrupt RED binding to SMU1.

Thus, the present invention also relates to a process for high-throughput pharmacological screening (HTS) of compounds which disrupt and/or destabilize the RED-SMU1 complex, comprising:
a) mixing a test compound, with a peptide comprising at least the sequence SEQ ID NO:3. Preferably, step a) comprises mixing a test compound, with the peptide consisting of SEQ ID NO:3 or SEQ ID NO:5 (SMU1_{Nter} 1-196 or 1-202 respectively);
b) adding a compound of formula (A) (including formulas (A'), (I) and (I')) conjugated to a fluorophore to the mixture obtained in a). Preferably, the compound of formula (A) (including formulas (A'), (I) and (I')) conjugated to a fluorophore is a compound of formula (I) in which R3 is -NH-(CH2)m-triazole-fluorophore; and
c) measuring the binding of the conjugated compound in the mixture obtained in step b),
wherein if the binding measured in step c) is less than the binding measured in a control, then concluding that the test compound disrupts and/or destabilizes the RED-SMU1 complex.

In another embodiment, typically, step a) comprises mixing a compound of formula (A) (including formulas (A'), (I) and (I')) conjugated to a fluorophore, with a peptide comprising at least the sequence SEQ ID NO:3; and step b) comprises adding a test compound to the mixture obtained in a).

The control is preferably a mixture comprising the same compound of formula (A) (including formulas (A'), (I) and (I')) conjugated to a fluorophore as the one of step b), and the same peptide comprising at least the sequence SEQ ID NO:3 as the one of step a).

The test compound may be, for example, a small molecule, a peptide, or a peptidomimetic.

Finally, the compounds of formula (A) (including formulas (A'), (I) and (I')) according to the invention may also be useful for other *in vitro* purposes, typically for imaging.

This is especially true when said compound is conjugated to a fluorophore, and/or when R3 is -NH-(CH2)m-triazole-fluorophore.

The figures used in the present application are the following and serve as illustrative purposes only:

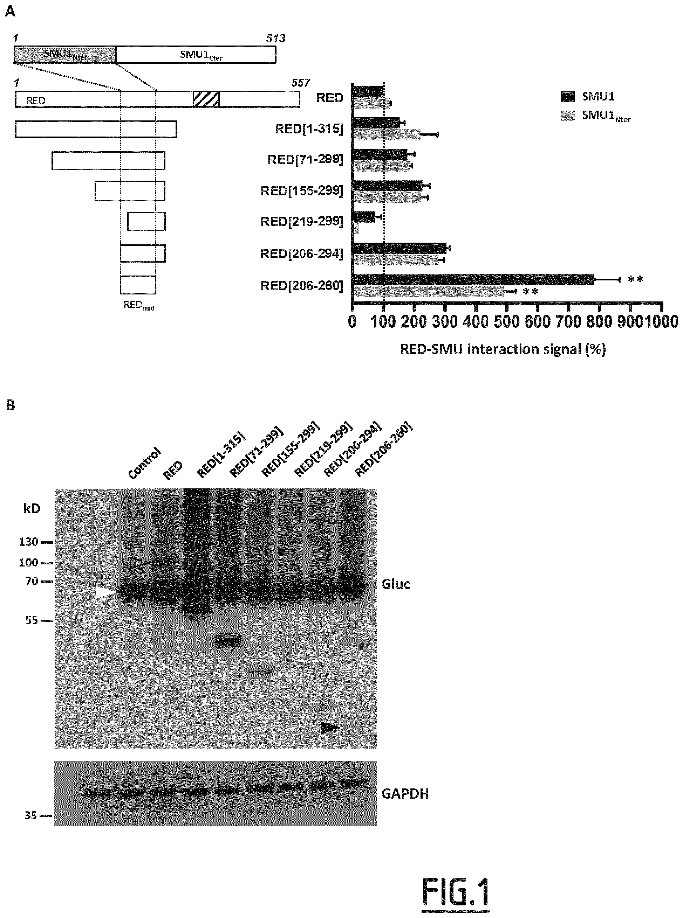

**Figure 1****. RED-SMU1 interaction mapping using a cell-based assay.**
   The *Gaussia princeps* luciferase-based complementation assay was performed as described in the Methods section. Gluc2-fused RED full-length (RED) or indicated subdomains were co-expressed with Gluc1-fused SMU1 full-length (SMU1) or SMU1[1-196] (SMU1_{Nter}) by transient transfection in HEK-293T cells. **A.** The normalized luciferase activities are expressed as percentages relative to the activity measured with full-length RED and SMU1 proteins. The data shown are the mean +/- SD of three independent experiments in triplicates. Black and grey bars represent luciferase activities measured in the presence of SMU1 and SMU1_{Nter}, respectively. ** : p<0.01 (parametric paired t-test). On the schematic diagram of RED its characteristic stretch of repeated arginine, glutamic acid and aspartic acid residues is represented by a hatched box. The dotted line is highlighting the interaction between the two components of the minimal SMU1_{Nter}-RED_{mid} complex. **B.** Cell lysates used to determine luciferase activities in A were subsequently analysed by western blot using antibodies specific for *Gaussia princeps* luciferase (Gluc, upper panel) and Glyceraldehyde phosphate deshydrogenase (GAPDH, lower panel). White arrowhead: Gluc1-SMU1; Opened arrowhead: Gluc2-RED; Black arrowhead: Gluc2-RED[206-260], renamed Glu2-RED_{mid} in the text.

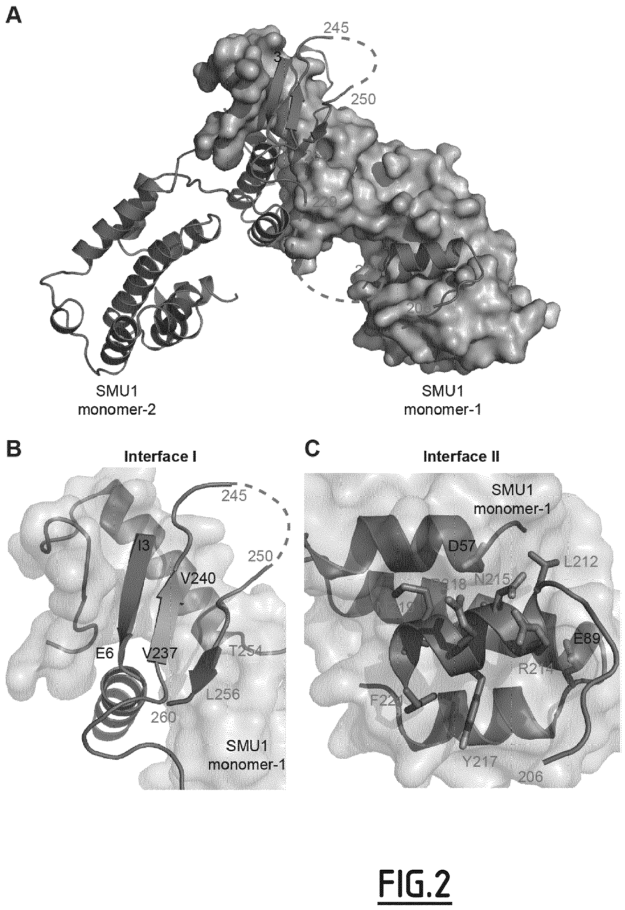

**Figure 2****: Crystal structure** of **the recombinant human RED_{mid}-SMU1_{Nter} complex.**
   **A.** Global view of the complex. The RED_{mid} and SMU1_{Nter} proteins are coloured in red and yellow respectively. One monomer of SMU1_{Nter} is shown as a ribbon diagram and the second as a surface representation. **B.** Close-up view of the mixed ß-sheet formed by the interaction of RED[235-257] with the N-terminal ß-strand of a SMU1_{Nter} monomer, and stabilized by the C-terminal surface of the other dimer partner SMU1_{Nter} molecule. **C.** Details of the second interface corresponding to the α-helical part of RED_{mid} inserted into a hydrophobic groove at the surface of SMU1_{Nter}. The images were generated using PyMOL (77).

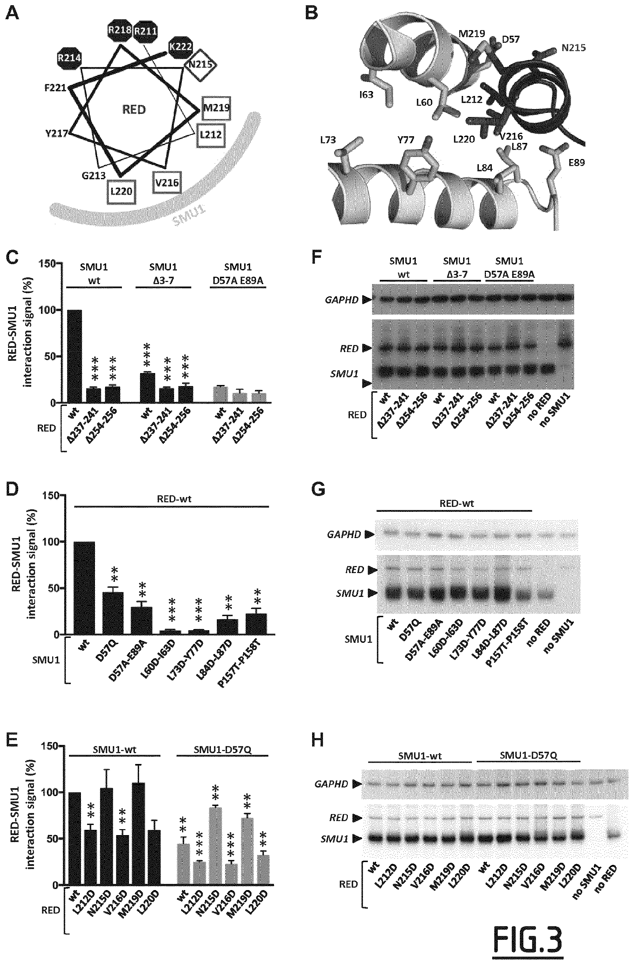

**Figure 3****. Structure-based analysis of the RED-SMU1 interface**
   **A.** Schematic representation of Interface II. On the helical weel representation of RED (residues 213 to 222), aliphatic residues (in a grey frame) are concentrated at the RED-SMU1 interface. The polar residues (in blue) are exclusively located on the opposite side. **B.** Location of SMU1 and RED residues (coloured in green and purple, respectively) involved in interactions at interface II and subjected to mutagenesis experiments shown in panels C to H. **C-E.** Luciferase-based complementation assays were performed as described in the Methods section with the indicated combinations of wild-type (wt) or mutant proteins. The normalized luciferase activities are expressed as percentages relative to the activity measured in the presence of the wt SMU1 and RED proteins. The data shown are the mean +/- SD of three independent experiments in triplicates, except when SMU1-D571-E89A was tested in (A): two independent experiments. ** p<0.01, *** p<0.001 unpaired t-test. **F.** The indicated combinations of wt or mutant SMU1 and RED proteins, fused to the Strep-tag, were transiently co-expressed in HEK-293T cells. Cell lysates were analysed by western blot, using HRP-conjugated Streptactin (lower panel). GAPDH (upper panel) was used as a loading control. **G-H.** Cell lysates used to determine luciferase activities in C and D were subsequently analysed by western blot, using antibodies specific for Gluc (lower panel) and an anti-GAPDH antibody (upper panel).
Figure **4****. SMU1_{Nter}** binding **to** compounds **LSP641 and LSP61**
   **A.** Flow chart of *in silico* screening and compound evaluation. The indicated assays were performed as described in the Methods section, in the presence of compounds at 60 µM or DMSO. The Venn diagrams show the number of selected compounds. The cut-off applied are indicated in italics (cell viability assay: < 2-fold reduction in CellTiter-Glo signal after 36h incubation with the compound compared to DMSO; RED-SMU1 and IAV replication assays: ≥ 20% and ≥ 80% reduction in Gaussia luciferase and Nanoluc signal, respectively, in the presence of the compound compared to DMSO). **B.** SMU1_{Nter} residues required for efficient binding to RED (coloured in green) and used for filtering of the docking poses. **C.** Chemical structures of compounds LSP641 and LSP61. **D.** Co-crystal structure of LSP641 in complex with SMU1_{Nter}.The LSP641 binding pocket is coloured according to hydrophobicity (blue: hydrophilic, red: hydrophobic). **E.** Residues of SMU1 involved in LSP641 binding. Green: key residues for RED binding which also interact with LSP641. Orange: other residues which strongly interact with LSP641 in the co-crystal structure. **F.** Representative pose of LSP61 upon *in silico* docking on SMU_{Nter}. The LSP61 binding pocket is coloured according to hydrophobicity (blue: hydrophobic, red: hydrophilic). **G.** Residues of SMU1 predictively involved in LSP61 binding. Green: key residues for RED binding which also interact with LSP61 upon molecular docking. Orange: other residues which strongly interact with LSP61 upon molecular docking.
**Figure 5****. Evaluation of compound LSP61 for RED-SMU1 destabilisation.**
   **A.** RED-SMU1 interaction assay. The split-luciferase assay was performed as described in the Methods section. At 8 hpt, the A549 cells were incubated with the relevant compounds at the concentrations indicated. The normalized luciferase units measured at 24 hpt are expressed as percentages relative to the DMSO-treated control. The data shown are the mean +/- SD of three independent experiments in triplicates. ** p<0.005 (parametric paired t-test). **B.** The split luciferase interaction assay was performed as in A, using Gluc1-FOS and Gluc2-JUN plasmids. The data shown are the mean +/- SD of two independent experiments in triplicates. **C.** Effect on the steady-state levels of the endogenous RED and SMU1 proteins. A549 cells were incubated with the indicated concentrations of LSP61 (+) or with DMSO (-) for 24h. Total cell extracts were analyzed by western blot, using antibodies specific for RED, SMU1 and GAPDH. **D.** Cell viability assay. A549 cells were incubated with the indicated concentrations of LSP61 or with DMSO. ATP levels, which reflect the number of viable cells, were determined using the CellTiter-Glo kit (Promega) at the onset of the experiment (T0), and following 24 or 48 hours of incubation (T24, T48). The data shown are the mean of luciferase units +/- SD of two independent experiments in duplicates.
**Figure 6****. Evaluation of compound LSP61 for anti-IAV activity**
   **A.** Effect on IAV replication. A549 cells were infected with the WSN-Nanoluc or RSV-Firefly virus (0.001 and 0.01 PFU/cell, respectively) and were incubated with LSP641 or LSP61 at the indicated concentrations, or with DMSO. The luciferase units measured at 24 hpi are expressed as percentages relative to DMSO-treated control. The data shown are the mean +/- SD of three (WSN-Nanoluc) or two (RSV-Iuc) independent experiments in triplicates. **p<0.005, ***p<0.0001 (parametric paired t-test). **B-C.** Effect on the production of infectious particles. MDCK-SIAT (B) or A549 cells (C) were infected with the WSN, H1N1pdm09 or H3N2 viruses as indicated, and were incubated with 60 µM of LSP61 or with DMSO. At 24, 48, and 72 hpi, the supernatants were collected and viral titers were determined by plaque assay. The data are expressed as mean +/- SD of three independent experiments (except for the 24 hpi time-point in C and D : two independent experiments), each in triplicates that were pooled for titration. * p<0.05, ** p<0.01, *** p<0.001, parametric paired t-test. **D.** Effect on NS1 mRNA splicing. A549 cells were infected with the WSN-wt virus (5 PFU/cell), and incubated with 60 µM of LSP641 or LSP61, or with DMSO. After 6 h incubation, polyA+ RNA were extracted and the levels of NS2 and NS1 mRNAs were analyzed by quantitative real-time PCR and normalized with respect to GAPDH mRNA levels. The NS2/NS1 mRNA ratios shown are the mean +/- SD of three independent experiments in duplicates. p=0.01, parametric paired t-test.

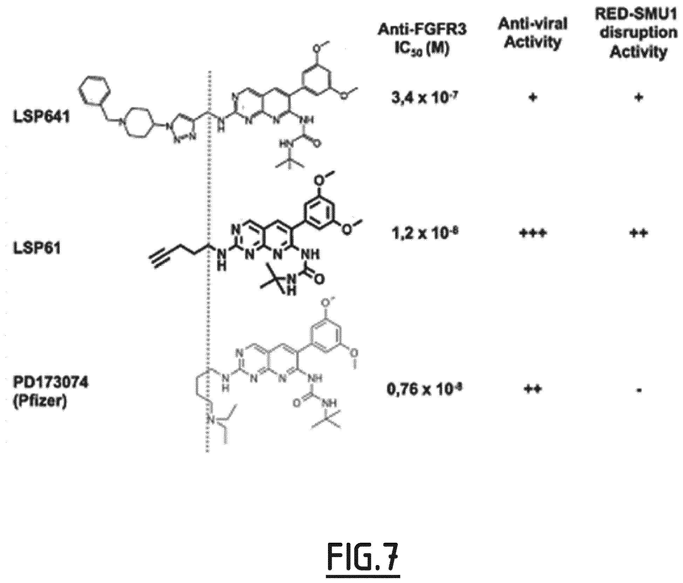

**Figure 7****. Comparison of compounds LSP641, LSP61 and PD173074.**
   Compounds LSP641 and LSP61 were compared to the compound PD173074 (prior art) in terms of (i) anti-FGFR3 activity: the IC50 was determined by the Reaction Biology company. Enzymatic inhibition was determined measuring the processing of 33P-ATP by recombinant FGFR3, (http://www.reactionbiology.com/webapps/site/KinasePDFs/FGFR3.pdf), (ii) anti-viral activity as assessed using the WSN-PB2-Nanoluc reporter virus and (iii) RED-SMU1 disruption activity as assessed using the cell-based split-luciferase complementation assay.
**Figure 8****. Strategy for chemical optimization.**
   A. Synthesis of fluoresceine-conjugated LSP61 derivatives.
   B. Schematic representation of the LSP61-based high-throughput pharmacological assay.

### Supplementary Figure S1. X-ray structure of SMU1_{Nter}.

The X-ray structures of SMU1_{Nter} (**A**) and RED_{mid}-SMU1_{Nter} (**B**) have been superimposed according to their dimerization interface. The two monomers of SMU1_{Nter} are coloured yellow and bright orange. RED_{mid} is coloured red in panel B.

### Supplementary Figure S2. Common RED-SMU1 interface (interface II) between H. sapiens and C. elegans structures.

**A.** Structure of the RED-SMU1 interface within the *C. elegans* RED[194-221]-SMUI_{Nter} complex (Ulrich AKC, Schulz JF, Kamprad A, Schutze T, & Wahl MC (2016) Structural Basis for the Functional Coupling of the Alternative Splicing Factors Smu1 and RED. Structure 24(5):762-773) compared with the corresponding interface (interface II) within the human RED_{mid}-SMU1_{Nter} complex (this study). The human structure was superimposed with *C. elegans* structure (PDB code 5EN7) with a root mean square deviation (r.m.s.d.) of 0.97 Å (112 Cα of SMU1_{Nter} aligned) using PDBeFold (Krissinel E & Henrick K (2004) Secondary-structure matching (SSM), a new tool for fast protein structure alignment in three dimensions. Acta Crystallogr D Biol Crystallogr 60(Pt 12 Pt 1):2256-2268). The two X-ray structures are shown in the same orientation and colour coded as in Figure 2. **B-C.** Partial sequence alignment of RED (SEQ ID NO:1 and 2) (B) and SMU1 (SEQ ID NO:3 and 4) (C). Identical residues are white on a red background and similar residues are red in a blue box. B. The boundaries and secondary structures of the RED region present in the human structure (residues 206 to 260, Uniprot ID Q13123; SEQ ID NO:1) and *C. elegans* structure (residues 163 to 223, Uniprot ID Q9N4U5; SEQ ID NO:2) are represented schematically above and below the alignment, respectively. C. The boundaries and secondary structures of the SMU1_{Nter} region present in the human structure of SEQ ID NO:3 (residues 1 to 196, Uniprot ID A0MNN4) and *C. elegans* structure (residues 1 to 181, Uniprot ID G5EEG7; SEQ ID NO:4) are represented schematically above and below the alignment, respectively.

### Supplementary Figure S3. Dimerization status of the full-length SMU1 protein.

**A.** SEC-MALLS-RI analysis. The Size Exclusion Chromatography (SEC), MALLS (Multi Angle Lase Light Scattering) and Refractive Index (RI) analyses were performed as described in the SI Appendix Materials and Methods section. Experimental determination of the Molecular weight (Mw) of the full-length SMU1 protein is shown (the theoretical Mw is 57.8 kDa for a SMU1 monomer). The curve was generated with Graphpad (Prism). **B.** Cross-linking of the SMU1 dimer in living cells. Western blot analysis was performed on lysates from A549 (left panels) or HEK-293T cells (rights panels) with or without pretreatment with the membrane permeable cross-linking agents DSS at the indicated concentrations. The membrane was first probed with anti-SMU1 antibodies (upper panels), then with anti-GAPDH antibodies (lower panels). (-): DMSO control.

### Supplementary Figure S4. Effect of RED_{mid} overexpression

**A.** RED_{mid}-SMU1 interaction in the absence or presence of the V216D mutation on RED_{mid}. HEK-293T cells were transfected with Gluc1-SMU1 together with the Gluc2-RED, -RED-V216D, -RED_{mid} or RED_{mid}-V216D plasmids. The normalized luciferase activities were expressed as percentages relative to the activity measured in the presence of Gluc2-RED. The data shown are the mean +/- SD of three independent experiments in triplicates. **B-F.** Effect of RED_{mid} overexpression. **B.** Effect on RED_{mid}-SMU1 interaction. The split-luciferase RED-SMU1 interaction assay was performed as described in the Methods section, in the presence of a co-transfected mCherry-RED_{mid} or control mCherry-RED_{mid}-V216D plasmids (100 ng). The normalized luciferase activities were expressed as percentages relative to the activity measured in the mock pCI-transfected control. The data shown are the mean +/- SD of three independent experiments in triplicates. ** : p<0.01 (parametric paired t-test). **C.** HEK-293T cells were transfected with 100 ng of pCI, mCherry-RED_{mid} or mCherry-RED_{mid}-V216D plasmids. At 48 hpt, the mCherry fluorescence signals were observed using a fluorescence microscope (Olympus CKX41). The data shown are representative of two independent experiments. **D.** Effect on the steady-state levels of the endogenous RED and SMU1 proteins. HEK-293T cells were transfected with an expression plasmid coding for RED_{mid} or RED_{mid}-V216D mutant fused to mCherry, or with the control plasmids pCI / pCI-mCherry (mC). Total cell extracts were prepared at 48 hours post-transfection (hpt) and were analysed by western blot, using antibodies specific for RED, SMU1 and GAPDH. One representative experiment out of three is shown. **E.** Effect on IAV replication. HEK-293T cells were transfected as in A with the indicated plasmids and infected at 48 hpt with the WSN-Nanoluc virus at a MOI of 0.001 PFU/cell. The luciferase activities measured at 24 hpi are expressed as percentages relative to the pCI-transfected control. The data shown are the mean +/- SD of three independent experiments in triplicates. **F.** Effect on NS1 mRNA splicing. HEK-293T cells were transfected as in A with the indicated plasmids and infected at 48 hpt with the WSN virus at a MOI of 5 PFU/cell. At 6 hpi, polyA+ RNA were extracted and the levels of NS2 and NS1 mRNAs were analyzed by quantitative real-time PCR and normalized with respect to GAPDH mRNA levels. The NS2/NS1 mRNA ratios shown are the mean +/-SD of three independent experiments in duplicates. **p<0.05, ***p<0.001, unpaired t-test (A-B), paired t-test (E-F).

**Supplementary Figure S5. Effect of incubation with LSP61 on cell monolayers.** Monolayers of A549 (A) or MDCK-SIAT (B) cells were incubated with the indicated concentrations of LSP61 or DMSO alone. They were imaged at 10x magnification at the onset of the experiment (T0), and following 48 hours of incubation (T48).

**Supplementary Figure S6. Effects of compounds LSP641 and LSP61 on splicing of the viral M1 and PB2 mRNAs. A.** M1 mRNA splicing. The same RNA samples that were tested for NS1 mRNA splicing in Figure 6D were used. The levels of M2 and M1 mRNAs were analyzed by RT-qPCR and normalized with respect to GAPDH mRNA levels, as described in Fournier G, *et al.* (2014) Recruitment of RED-SMU1 complex by Influenza A Virus RNA polymerase to control Viral mRNA splicing. *PLoS Pathog* 10(6):e1004164. The M2/M1 mRNA ratios shown are the mean +/- SD of three independent experiments in duplicates. ns : non significant, unpaired t-test. **B.** PB2 mRNA splicing. The same RNA samples that were tested for NS1 and M1 splicing were used. The levels of PB2 and PB2-S1 mRNAs were analyzed by semi-quantitative RT-PCR as described in Yamayoshi S, Watanabe M, Goto H, & Kawaoka Y (2016) Identification of a Novel Viral Protein Expressed from the PB2 Segment of Influenza A Virus. J Virol 90(1):444-456. The results representative of one experiment out of three are shown.

**Supplementary Figure S7. Refined spliceosome complex obtained from cryo-EM structure (PDB ID:5O9Z) superimposed to RED_{mid}-SMU1 co-crystal (this study).** Side view **(A)** and front view after a 90° rotation around the horizontal axis **(B)** of the spliceosome complex showing the pivotal position of SMU1 dimer (blue) and RED (red) in the spliceosome complex.

### Example 1: Destabilisation of the human RED-SMU1 splicing complex as a basis for host-directed anti-influenza therapy

### Materials and Methods

### Cells, viruses, plasmids and reagents:

### Cells

Human embryonic kidney (HEK-293T) and adenocarcinomic human alveolar basal epithelial (A549) cells were obtained from Dr. M. Perricaudet (CNRS-IGR, Villejuif, France) and Pr. M. Schwemmle (Freiburg University), respectively, and were grown in Dulbecco's modified Eagle's medium (DMEM) supplemented with 10% heat-inactivated fetal calf serum (FCS). Madin Darby Canine Kidney cells stably overexpressing the human 2,6-sialtransferase (MDCK-SIAT1, Matrosovich M, Matrosovich T, Carr J, Roberts NA, & Klenk HD (2003) Overexpression of the alpha-2,6-sialyltransferase in MDCK cells increases influenza virus sensitivity to neuraminidase inhibitors. J Virol 77(15):8418-8425) were obtained from Pr. M. Matrosovich (Marburg University) and were grown in minimum essential Eagle's medium (MEM) supplemented with 5% FCS.

### Viruses

The recombinant influenza A/WSN/33 (WSN) and WSN-PB2-Nanoluc viruses were described previously in Diot C, *et al.* (2016) Influenza A Virus Polymerase Recruits the RNA Helicase DDX19 to Promote the Nuclear Export of Viral mRNAs. *Sci Rep* 6:33763. Human seasonal IAVs A/Bretagne/7608/2009 (H1N1pdm09) and A/Paris/1003/2012 (H3N2) were provided by the National Influenza Center at the Institut Pasteur (Paris, France). The recombinant respiratory syncytial virus (RSV-Firefly) was described previously in Rameix-Welti MA, et al. (2014) Visualizing the replication of respiratory syncytial virus in cells and in living mice. Nat Commun 5:5104. The recombinant WSN-PB2-Nanoluc or RSV-Firefly virus were used at a m.o.i. of 0.001 or 0.01 PFU/cell, respectively. Following viral adsorption for 2 hours, the inoculum was replaced with medium supplemented with drug or DMSO alone, and luciferase activities were determined 24 hours later using *Nano-Glo* luciferase or Firefly luciferase assay kits (Promega) and a Berthold Centro XS luminometer. The WSN, A/Bretagne/7608/2009, or A/Centre/1003/2012 virus were used at a MOI of 0.0001 (WSN) or 0.001 PFU/cell. Following viral adsorption for 2 hours the inoculum was replaced with DMEM supplemented with drug or DMSO alone, and with 0.5 µg/ml TPCK-treated trypsin (Sigma-Aldrich). At 24, 48, and 72 hpi (hours post-infection), cell supernatants were harvested and infectious titers determined using a plaque assay on confluent monolayers of MDCK-SIAT1 cells.

### Plasmids for split-luciferase-based interaction assays

The pCl-Gluc2-RED and pCl-Gluc1-SMU1 plasmids were described previously in Fournier G, et al. (2014) Recruitment of RED-SMU1 complex by Influenza A Virus RNA polymerase to control Viral mRNA splicing. PLoS Pathog 10(6):e1004164. Sequences encoding RED/SMU1 subdomains were amplified by PCR, and the resulting amplicons were cloned in place of RED/SMU1 into the pCl-Gluc2-RED/pCl-Gluc1-SMU1 plasmids using the NotI and MluI restriction sites. The mCherry-RED-[206-260] plasmid was obtained by cloning the Cherry sequence in place of Gluc2 into the pCl-Gluc2-RED-[206-260] plasmid using the XhoI and NotI restriction sites. The pCl-Gluc1-FOS and pCl-Gluc2-JUN plasmids were obtained by Gateway cloning using the LR Clonase enzyme mix (Thermo Fisher Scientific), donor plasmids from the human ORFeome v8.1 library (http://horfdb.dfci.harvard.edu) and destination vectors described in Cassonnet P, et al. (2011) Benchmarking a luciferase complementation assay for detecting protein complexes. Nat Methods 8(12):990-992. Point mutations and deletions were introduced using the QuikChange II Site-Directed Mutagenesis Kit (Agilent). All constructs were verified by Sanger sequencing. The sequences of the oligonucleotides used for amplification, mutagenesis and sequencing can be provided upon request.

### Plasmids for protein expression in E. coli

The E. *coli* codon-optimized full-length human SMU1 and RED genes were synthesized by GeneArt (Life Technologies). The coding sequence of RED_{mid} (residues 206 to 260) was PCR amplified and cloned in pETM11 (EMBL) to express an N-terminal His-tagged (TEV cleavable) recombinant construct. The coding sequence of SMU1_{Nter} (residues 1 to 196) was PCR amplified and cloned in both pETM11 and pACYC-LIC+ (Addgene) to express respectively the N-terminal His-tagged (TEV cleavable) and non His-tagged recombinant construct. The pACYC-LIC+- SMU1_{Nter} plasmid was used to transform *E. coli* BL21 cells (Life Technologies) and prepare the resulting chemical competent cells (*E*. *coli* BL21-RIL-SMU1_{Nter}) grown in LB containing chloramphenicol (34 µg.mL⁻¹).

### Expression, purification and characterization of full-length SMU1

The codon-optimized full-length human SMU1 gene was cloned in pFastBac-HtB (Thermo Fisher Scientific) to express the N-terminal His-tagged (TEV cleavable) recombinant construct in insect cells (High Five cells; Thermo Fisher Scientific) using the Bac-to-Bac baculovirus system (Thermo Fisher Scientific) in standard conditions. Cells were resuspended in lysis buffer (50mM Tris-HCI pH 8.0, 200 mM NaCl, 1 mM β-mercaptoethanol) and cryolysed. After centrifugation, the supernatant was complemented with imidazol to reach 25 mM and load on a nickel affinity column (NiNTA, GIAGEN). The resin was washed with a high salt buffer (50 mM Tris-HCI pH 8.0, 1 M NaCl, 1 mM β-mercaptoethanol, 25 mM Imidazol). The purified protein was then eluted with 50 mM Tris-HCI pH 8.0, 200 mM NaCl, 1 mM β-mercaptoethanol, 300 mM Imidazol. The protein was dialyzed with TEV protease overnight against buffer without Imidazol. The protein sample was loaded on a second NiNTA, concentrated and loaded on Superdex S200 (GE Healthcare) with the running buffer containing 20 mM Tris-HCI pH 8.0, 150 mM NaCl; 5 mM β-mercaptoethanol. The SEC-MALLS-RI analysis was performed using a Superdex™ 200 increase column (10/300 GL, GE Healthcare). Sample injection and buffer flow was controlled by a Hitachi L2130 pump, following the SEC column was a L-2400 UV detector (Hitachi), Optilab T-rEX refractometer (Wyatt technologies) and a DAWN HELEOS-II multi angle light scattering detector (Wyatt technologies). Prior to injection, column and system were equilibrated in 5 to 10 column volumes of the running buffer (20 mM Tris-HCI pH 8.0, 150 mM NaCl; 5 mM β-mercaptoethanol). 50 µL of the protein sample concentrated at 3.5 mg.mL⁻¹ injection was injected at a constant flow rate of 0.5 mL.min⁻¹. Accurate MALLS mass prediction was performed with the Astra software (Wyatt Technologies).

### Reagents

Western blots were performed as described earlier in Moisy D, et al. (2012) HMGB1 protein binds to influenza virus nucleoprotein and promotes viral replication. Journal of virology 86(17):9122-9133. The membranes were incubated with primary antibodies directed against RED (Santa Cruz, sc-135485) or SMU1 (Santa Cruz, sc-100896), GAPDH (Thermo Fisher Scientific, MA5-15738), the Gaussia luciferase (New England Biolabs, E8923S), with peroxidase-conjugated Streptavidin (IBA) and peroxidase-conjugated secondary antibodies (GE Healthcare). Western blot signals were revealed using the enhanced chemiluminescent (ECL) reagent (Pierce). For intra-cellular protein cross-linking, cell monolayers were washed twice with PBS and treated with using disuccinimidyl suberate (Thermo Fisher Scientific, #A39267) at the indicated concentrations for 30 min at room temperature. The reaction was stopped by adding a quenching solution (Tris-HCI 1M, pH 7.5) for 15 mn at room temperature. Cell lysates were prepared using a RIPA buffer and were subjected to western blot analysis.

Cell viability was assessed using the CellTiter-Glo® One Solution Assay kit (Promega) according to the manufacturer's instruction.

### Assessment of chemical database diversity

The diversity of our in-house chemical database was computed using the Calculate Diversity Metrics protocol (Discovery Studio Modeling Environment, release 4.5; Dassault Systemes BIOVIA: San Diego, 2015) and compared with the Prestwick and FDA approved databases. All three databases were similar in terms of diversity number of assemblies (LCPBT-DB: 0.341; FDA: 0.300; Prestwick: 0.468), diversity number of fingerprint features (LCPBT-DB: 8.1; FDA: 5.8; Prestwick: 11.2), and normalized fingerprint distances (LCPBT-DB: 0.910; FDA: 0.906; Prestwick: 0.913). The number of assemblies is the number of unique Ring, Chain, Bridge, or Murcko groups normalized by the number of molecules. Murcko groups are contiguous ring systems plus chains that link two or more rings, as defined by Bemis GW & Murcko MA (1996) The properties of known drugs. 1. Molecular frameworks. J Med Chem 39(15):2887-2893. The number of fingerprints is measured by the number of unique fingerprint features of the selected fingerprint property normalized by the number of molecules. Fingerprint distances report the minimum, maximum, and average fingerprint distance for all pairs of ligands in the set. Chemical compounds were provided by the chemical library of LCPBT (Laboratoire de Chimie, Biochimie Pharmacologiques et Toxicologiques - UMR8601), and compounds purity was >80%.

### In cellulo split luciferase-based interaction assays

The split-luciferase protein complementation assays were performed as described in (65). Briefly, HEK-293T or A549 cells were seeded in 96-well white opaque plates (Greiner Bio-One) and co-transfected with 100 ng of each indicated pGluc1-P1 and pGluc2-P2 plasmids, where P1 and P2 represent proteins or protein subdomains of interest. The polyethylenimine PEI (Polysciences Inc) and JetPRIME (Polyplus Transfection) transfection reagents were used with HEK-293T and A549 cells, respectively. Normalized luciferase values were determined as described in (23, 65). At 24 h post-transfection (24hpt), the luciferase enzymatic activities were measured using the *Renilla* luciferase assay system (Promega) and a Berthold Centro XS luminometer. When cell-based interaction assays were performed for compound screening, control HEK-293T cells transfected with a full-length Gaussia luciferase expression plasmid were incubated in parallel with tested compounds or DMSO alone. The split-luciferase values were normalized with respect to control full-length luciferase signals.

### Protein expression in E. coli and protein purification

The pETM11-SMU1_{Nter} plasmid was transformed in *E. coli* BL21-CodonPlus (Agilent). Cultures were grown at 37°C in LB containing kanamycin (30 µg/mL) and chloramphenicol (34 µg/mL). When the OD₆₀₀ₙₘ reached 0.6-0.8, the cultures were cooled down to 18°C and expression induced with 0.3 mM isopropyl-thiogalactopyranose (IPTG). The cultures were incubated overnight at 18°C before centrifugation. For the SMU1_{Nter}-RED_{mid} complex, a co-expression strategy was set up. The pETM11-RED_{mid} plasmid was transformed in E. *coli* BL21-RIL-SMU1_{Nter} competent cells and expression performed using the protocol described above.Cell pellets were resuspended in lysis buffer (50 mM Hepes pH7.5, 150-500 mM NaCl, 1 mM β-mercaptoethanol (βme)), and sonicated. After centrifugation, the supernatant was complemented with imidazol to reach 25mM and loaded on a nickel affinity column (NiNTA, GIAGEN). The resin was washed with a high salt buffer (50 mM Hepes pH7.5, 1 M NaCl,1 mM βme). The recombinant proteins were eluted with elution buffer (50 mM Hepes pH7.5, 1 M NaCl, 1 mM βme, 300mM imidazole). Proteins were dialyzed with TEV protease overnight against the buffer without imidazole, loaded on a second NiNTA column, concentrated and loaded on a size exclusion column, a HiLoad 16/600 Superdex 75 (GE Healthcare) for SMU1_{Nter} or a Superdex 200 Increase 10/300GL (GE Healthcare) for the SMU1_{Nter}-RED_{mid} complex. Fractions of interest were concentrated between 5 and 15 mg/mL.

### Structure determination

For SMU1_{Nter}, the crystals (native and Se-Met derivative) were obtained in 0.1 M Bis-Tris pH 5.5, 16-20 % PEG 10K, 0.1 M ammonium acetate and cryoprotected in the same solution + 30 % glycerol. Data were processed with the XDS package (66). For experimental phasing of SMU1_{Nter} a highly redundant single wavelength anomalous dataset of a Se-Methionine (Se-Met) derived crystal was collected to 2.1 Å resolution at the peak of the Se-Met signal, as measured by X-ray fluorescence, on ID29 (67) at the European Synchrotron Radiation Facility (ESRF). For structural solution 8 Se-Met sites were located on the basis of their anomalous differences using SHELXC/D/E (68). These sites were subsequently refined and experimental phases were calculated using the single anomalous dispersion (SAD) procedure in SHARP (69). These phases were further improved by density modification followed by model building with the Buccaneer software (70). Model building and refinement were performed using CCP4i suite program for crystallography (PHASER, ARP/wARP, REFMAC5, COOT) (71-75)

For the RED_{mid}-SMU1 _{Nter} complex, the crystals (native and Se-Met derivative) were obtained in 0.1 M Hepes pH 7.0-7.5, 8-10 % PEG 8K and cryoprotected in the same solution + 30 % glycerol. Data were processed with XDS and the structure was solved by molecular replacement using the native SMU1_{Nter}.structure. The model was built using the anomalous signal of a Se-met derivative dataset for an accurate side chains attribution, and the final refinement was performed using the software mentioned above.

For the SMU1_{Nter} + LSP641 complex, a mother solution of LSP641 was prepared at 125 mM in DMSO. The co-crystals of SMU1_{Nter} (10 mg/mL) + 2.5 mM LSP641 were obtained in 0.1 M Bis-Tris pH 6, 16-20 % PEG 10K, 0.2 M ammonium acetate and cryoprotected in the same solution + 30 % glycerol. Data collection, processing and model building were performed as described above.

### In silico screening

Docking experiments were performed using the LibDock protocol, as implemented in Discovery Studio (Discovery Studio Modeling Environment, release 4.5; Dassault Systemes BIOVIA: San Diego, 2015), an interface to the LibDock program developed by Diller and Merz (76). LibDock uses protein site features referred to as HotSpots that fall into two categories: polar and apolar HotSpots. The receptor HotSpot file was calculated prior to the docking procedure. Random ligand conformations were generated from the initial ligand structure through high-temperature molecular dynamics using the BEST algorithm before docking. The rigid ligand poses were placed into the active site and HotSpots were matched as triplets. The poses were pruned and a final optimization step was performed before the poses were scored. Ligand hydrogens, which were removed during the docking process, were added back to the ligand poses and optimized by minimization. The poses with the highest LibDock scores were retained and clustered according to their binding mode.

### RNA extraction and reverse transcription-quantitative PCR

Total RNA and poly(A)+ RNAs were extracted subsequently using the RNeasy Mini Kit (Qiagen) and Oligotex mRNA Mini kit (Qiagen), following the manufacturer's protocol. Reverse transcription (RT) was performed on 5-10 ng of total mRNA (or poly(A)+ RNA equivalent) using the Maxima First Strand cDNA Synthesis Kit (Thermo Scientific). Quantitative real-time PCR was performed on 2 µl of a 1/10 dilution of the RT reaction using the Luminaris Color Probe qPCR Master Mix (Thermo Scientific) and a Light Cycler 480 (Roche). The levels of NS1/NS2 and M1/M2 mRNAs were determined and normalized with respect to GAPDH mRNA levels using the protocol described in (23). The levels of PB2/PB2-S1 mRNAs were determined by semi-quantitative RT-PCR as described in (35).

### Data Availability Statement

Structure coordinates and diffraction data are available on the Protein Data Bank (http://www.pdb.org) under accession codes PDB: 6Q8F (SMU1_{Nter}), 6Q8I (RED_{mid}-SMU1_{Nter}), and 6Q8J (SMU1_{Nter}-LSP641).

### Results

### Delineation of a minimal RED-SMU1 complex

The inventors previously found that the N-terminal domain of RED (residues 1 to 315) binds to SMU1 as efficiently as the full-length RED protein (23). Using a split-luciferase based interaction assay, the inventors further delineated a minimal human RED-SMU1 complex. Plasmids encoding various subdomains of RED tagged with the Gluc2 fragment of *Gaussia princeps* luciferase were co-transfected with plasmids for the expression of SMU1 or SMU1_{Nter} (residues 1-196) fused to the trans-complementing *Gaussia princeps* luciferase Gluc1 fragment. The rationale for RED truncations shown in **Fig. 1A** was based on secondary structure and disorder predictions (30), with an iterative testing and design approach. As revealed by the relative luciferase activities measured in cell extracts, the RED[206-260] subdomain (named thereafter RED_{mid}) retained the interaction with SMU1 (black bars) or SMU1_{Nter} (grey bars) (**Fig**. **1A**). The SMU1 interaction signals were almost 8-fold higher with RED_{mid} than with the full-length RED (p<0.01), and were higher than with any longer truncated version of RED. Western blot analysis of the cell lysates used for luciferase assay showed that this higher interaction signal was not due to a higher level of expression of RED_{mid} (**Fig**. **1B****,** black arrowhead) compared to other deletion mutants of RED or to the full-length RED (open arrowhead). A likely explanation is that in the context of RED, which is essentially an intrinsically disordered protein according to predictions, polypeptides adjacent to RED_{mid} make additional interactions with other domains of SMU1 and/or other cellular partners, thereby resulting in an overall lower binding to SMU1. For several other protein pairs, it was indeed previously shown that interactions are more easily detected when using isolated interactions domains or peptides (31). Notably, the RED[219-299] subdomain produced lower interaction signals compared to RED_{mid} but also to the full-length RED protein, suggesting that residues 206 to 218 of RED are essential for the interaction with SMU1.

### The 3D structure of human RED_{mid}-SMU1_{Nter} reveals two molecular interfaces

Based on these cell-based interaction results, the inventors set up a co-expression strategy to produce and pull-down the human RED_{mid}-SMU1_{Nter} complex, using a hexa-histidine tag fused to the N-terminus of RED_{mid}. The recombinant complex was crystallized and the structure solved by molecular replacement using the X-ray structure of the SMU1_{Nter} alone without RED_{mid} obtained earlier (**Fig. S1**).

The final model was built using the anomalous signal of a Se-met derivative dataset for an accurate sequence assignment. The asymmetric unit is composed of sixteen molecules: eight SMU1_{Nter} molecules that assemble into four dimers, and eight RED_{mid} molecules each one being associated with a SMU1_{Nter} monomer. The X-ray structure reveals two distinct interfaces between SMU1_{Nter} and RED_{mid} (**Fig. 2A**). First, the region of RED_{mid} corresponding to residues 235-257 disrupts the initial fold of the N-terminus of SMU1_{Nter} to form a three-stranded antiparallel β-sheet that is uniquely stabilized by main chain interactions (Interface I, **Fig. 2B**). Second, the helical region of RED_{mid} (corresponding to residues 211-221) lies in a hydrophobic groove delimited by three continuous α-helices (α4 to α6) on the surface of SMU1_{Nter} (Interface II, **Fig. 2C**). These structural findings are consistent with the high and low interaction signals measured with the SMU1-RED[206-260] and SMU1-RED[219-299] combinations, respectively (**Fig**. **1A**).
The comparison of the human RED_{mid}-SMU1_{Nter} structure with the recently published structure of a *C*. *elegans* minimal RED-SMU1 complex (29) is shown in **Fig. S2.** Importantly, this structure suggests that SMU1 needs to form a dimer to interact with RED, as Interface I extends across two distinct SMU1_{Nter} monomers. In agreement with this observation, SEC-MALLS analysis on purified full-length SMU1 protein showed that it is present mostly as a dimer (**Fig. S3A**), and treatment of A549 or HEK-293T cells with the cell-permeable crosslinking agent disuccinimidyl suberate (DSS) followed by western blotting revealed that the endogenous SMU1 protein forms dimer in living cells **(Fig. S3B).**

### Mutational analysis provides a rationale for targeting the α-helix:groove interface

To confirm these structural data and assess the druggability of the RED-SMU1 interface, the inventors performed structure-based mutational analysis of the full-length RED and SMU1 proteins. Point mutations were introduced at the interface I **(****Fig. 2B****)** or II **(****Fig. 2C** and **Fig. 3A-B****).** Split-luciferase complementation assays were performed and expression levels of the mutants were assessed **(****Fig. 3C-H****).** Short deletions in the domains of SMU1 (Δ3-7) or RED (Δ237-241 or Δ254-256) involved in interface I reduced the RED-SMU1 interaction signal up to 3-fold (**Fig. 3C**, black bars). In the presence of SMU1 mutations targeting the interface II (D57A-E89A, L60D-I63D, L73D-Y77D, L84D-L87D) or the hinge between interface I and II (P157T-P158T), a 2- to 10-fold reduction of the interaction signal was also observed (**Fig. 3D**). Notably, no cumulative effect was observed when deletions at interface I were combined with the SMU1 mutations D57A-E89A destabilizing interface II (**Fig. 3C**, grey bars), suggesting that both molecular interfaces are required to stabilize the RED-SMU1 complex. RED mutants L212D, V216D and L220D targeting interface II showed a 2-fold reduction of the interaction signals with SMU1 or SMU1-D57Q (**Fig. 3E**). In contrast the RED-N215D and M219D mutations had little effect on the interaction with SMU1 (**Fig. 3E**), as expected from the 3D structure **(****Fig. 3A-B****).** Together, these results confirm the structural findings, demonstrate that a mere disruption of interface II α-helix:groove impairs the stability of the RED-SMU1 complex, and point to SMU1 D57, L60, I63, L73, Y77, L84, L87 and E89 as being key residues for binding to RED.

### Disrupting the RED-SMU1 complex: proof-of-principle by overexpressing RED_{mid}

Based on the data presented above, the inventors reasonned that if overexpressed, the RED_{mid} helical domain should efficiently compete with the full-length RED protein for binding to the surface groove on SMU1, and thereby disrupt the RED-SMU1 complex. As a control, the inventors used a RED_{mid}-V216D mutant, which accumulates at the same levels as RED_{mid} but is strongly impaired for SMU1 binding. Of note, the effect of V216D mutation on SMU1 binding is much stronger in the context of RED_{mid} (> 99% reduction) compared to the full-length RED (about 50% reduction) (**Fig. S4A**). Upon overexpression of the mCherry-RED_{mid} fusion protein the inventors observed a >2-fold reduction of the RED-SMU1 interaction signal, as measured with the split-luciferase complementation assay. With the control mCherry-RED_{mid}-V216D mutant, no such reduction was observed (p<0.01, **Fig. S4B-C).**

The inventors then assessed the effect of RED_{mid} overexpression on the endogenous RED-SMU1 complex. As there is evidence that RED and SMU1 stabilize each other (23, 26, 27), RED-SMU1 disruption is expected to result in decreased steady-state levels of the proteins. The inventors observed a decrease in endogenous cellular levels of RED when overexpressing RED_{mid}, whereas empty vectors or RED_{mid}-V216D had no effect (**Fig. S4D**, upper panel). However, SMU1 levels remained unchanged (**Fig. S4D**, middle panel). A likely interpretation is that the SMU1 protein complexed with RED_{mid} remains stable, whereas the dissociated RED protein undergoes degradation.

Next the inventors monitored the effect of RED_{mid} overexpression on the replication of a recombinant A/WSN/33 virus carrying a luciferase reporter gene (WSN-PB2-Nanoluc). The luciferase activity measured in cell lysates prepared at 24 hours post-infection (hpi) was lower in cells overexpressing RED_{mid} compared to cells transfected with the empty pCI vector or overexpressing RED_{mid}-V216D (**Fig. S4E**). On the contrary, there was no significant difference between cells transfected with the empty pCI vector and those overexpressing RED_{mid}-V216D. Furthermore, splicing of the viral NS1 mRNA into NS2 mRNA, which is dependent upon RED-SMU1 (23), was reduced in RED_{mid}-expressing cells compared to the RED_{mid}-V216D control (**Fig. S4F**). Overall, these findings strengthen the rationale of targeting the RED-SMU1 α-helix:groove interface to inhibit IAV replication.

### In silico identification and evaluation of RED-SMU1 disrupting compounds

The inventors used the available structural information on the RED_{mid}-SMU1_{Nter} complex to perform molecular docking-based screening for compounds targeting the RED-binding groove at the surface of SMU1_{Nter}. The virtual high-throughput screening (vHTS) was performed on a set of 4,121 chemical compounds from the in-house chemical database (LCPBT-DB), comparable to the Prestwick or FDA-approved libraries in terms of chemical diversity. A flow chart for the vHTS and compound selection pipeline is represented in **Fig. 4A****.** To ensure a time/precision ratio compatible with vHTS the inventors used a protocol in which amino acid side chains of the protein are left flexible only around the binding site, and they tested 10 random conformers for each ligands. The resulting poses were ranked according to their docking scores, as described in the Methods section. The poses with the highest docking scores were further filtered to select for compounds that are predicted to bind at least 4 of the 8 SMU1 residues required for efficient binding to RED: D57, L60, I63, L73, Y77, L84, L87, E89 (**Fig. 4B** and **Fig. 3D**). A total of 37 compounds fulfilled both criteria, and a subset of 16 molecules with representative chemical scaffolds were further evaluated.

Among these, 14 showed no or only limited cytotoxicity at 60 µM, and were further tested at the same concentration in cell-based assays for the inhibition of RED-SMU1 interaction and IAV replication. Compounds inhibiting RED-SMU1 interaction by more than 20%, and IAV replication by more than 80% were selected for further evaluation. In total, three compounds showed promising inhibitory effect in both assays (**Fig. 4A**). Of these, one molecule, LSP641 (**Fig. 4C**), was successfully co-crystallised with the purified recombinant SMU1_{Nter} domain. Structural data showed that LSP641 locates in the hydrophobic RED binding pocket of SMU1 as expected (**Fig. 4D**) and is engaged in several molecular interactions with SMU1 (**Fig. 4E**). The 2-amino-pyrimidine group present in LSP641 forms key hydrogen bounds with Q61 and Q64, whereas the urea group forms a hydrogen bond with Y77. In the apolar region of SMU1, D57, L60 and L96 are involved in Pi-Pi or σ-Pi interactions, in particular with the pyridopyrimidine scaffold of LSP641. Furthermore, W56, I63, L84 and A92 form hydrophobic interactions with LSP641. Remarkably, the LSP641 molecule interacts with D57, L60, I63, Y77 and L84, *i.e.* 5 of the 8 residues shown earlier to be involved in RED-SMU1 interaction, to account for the inhibition of this protein-protein interaction.

Guided by the SMU1_{Nter}:LSP641 co-structure, the inventors selected 27 LSP641-related compounds and filtered them through a second evaluation round (**Fig. 4A**), which led to the selection of LSP61 (32) (Fig. **4C and 4F**)**.** Unfortunately, the inventors were unable to co-crystallize LSP61 with SMU1 _{Nter}. This different behavior of LSP61 compared to LSP641 might be related to the absence/presence of stacking interactions in the co-crystal. However, *in silico* molecular docking supported binding interactions similar to LSP641 (**Fig. 4G**). Hydrogen bonds with Q64 and Y77 are conserved, as well as Pi- or σ-Pi-interactions with D57 and L60, and hydrophobic interactions with W56 and L84. Q61 is involved in Pi-interactions, whereas V41, V80 and L96 are forming novel hydrophobic interactions. Most importantly, the benzylpiperidine group of LSP641 that is not binding SMU1 in the crystal structure (**Fig**. **4D-E****),** is replaced in LSP61 by an alkyl chain which interacts with A62 and V41 in a neighboring hydrophobic pocket of SMU1 and therefore optimizes molecular recognition (**Fig. 4F-G**). This led the inventors to select LSP61 to conduct further investigations, in parallel with LSP641.

LSP61 and LSP641 were compared in the RED-SMU1 interaction assay across the 0.23 to 60 µM concentration range. The EC50 (half-maximal effective concentration) was estimated to be 15 µM for LSP61; LSP641 showed a milder effect resulting in a 40% inhibition at 60 µM, compared to 90% with LSP61, and a plateauing curve (**Fig. 5A**, dark and light grey curves, respectively). When a similar FOS:JUN interaction assay was used as a specificity control, none of the two compounds showed any significant effect (Fig. **5B**). The Kd of the SMU1_{Nter}-LSP61 complex could not be determined, most likely due to the low solubility of LSP61 (logP=4.8). The effect of LSP61 on the endogenous RED-SMU1 complex was assessed using the steady-state levels of RED and SMU1 as a proxy. Expression levels of both proteins decreased when treating cells with 15, 30 and 60 µM LSP61 (**Fig. 5C**). At these concentrations, no cytotoxicity but some cytostatic effect was observed as assessed by the steady levels of ATP measured in culture wells (a proxy for cell count) upon 24 and 48 hours of incubation with LSP61 (**Fig**. **5D****)** and the observation of cell monolayers by bright field miscroscopy (**Fig. S5**).

### LSP61 inhibits IAV replication

Finally the inventors assessed the ability of LSP61 to inhibit IAV replication. The inventors first used the WSN-PB2-Nanoluc virus, in parallel with a recombinant human Respiratory Syncytial Virus (RSV) expressing Firefly luciferase as a specificity control (RSV replicates in the cytoplasm with no direct involvement of the splicing machinery postulated). After viral adsorption to A549 cells, the LSP61 or LSP641 compounds were added to the cells across the 7.5 - 60 µM concentration range, and the luciferase activities were determined at 24 hpi. The RSV-Firefly signals showed only moderate reductions (**Fig**. **6A****,** grey bars). In contrast the WSN-Nanoluc signals showed a significant, dose-dependent reduction, more pronounced for LSP61 (25-fold at 60 µM, p<0.0001) compared to LSP641 (6-fold at 60 µM, p<0.005) (**Fig**. **6A****,** black bars). Growth kinetics were performed with wild-type IAVs (the WSN virus and representatives of H1N1pdm09 and H3N2 circulating human IAVs) in the presence of 60 µM LSP61 and the supernatants were titrated by plaque assay. Because the H1N1pdm09 and H3N2 isolates grew poorly on A549 cells, canine MDCK-SIAT cells (33) were used in these experiments. In the presence of LSP61 the production of infectious particles showed a 10- to 100-fold reduction at 24 and 48 hpi (**Fig. 6B**). In comparison, on A549 cells, the production of WSN infectious particles was reduced about 1000-, 100- and 10-fold at 24, 48 and 72 hpi, respectively (**Fig. 6C**). In MDCK-SIAT cells, canine Mx proteins lack anti-influenza activity, which limits the establishment of a strong interferon-induced antiviral state and favors a very efficient replication of influenza viruses (34). This particular feature of MDCK-SIAT cells likely accounts for the fact that a strong inhibition with the LSP61 compound was more difficult to achieve compared to in A549 cells.

In WSN-infected cells, the LSP641 and LSP61 compounds inhibited the splicing of the viral NS1 mRNA into NS2 mRNA at 60 µM concentration. Indeed, the NS2 to NS1 mRNA ratio was reduced by 30% with LSP641, and 50% with LSP61 (**Fig. 6D**), which is comparable to previous results obtained when either RED or SMU1 was knocked-down in infected cells (23). In contrast LSP641 and LSP61 did not inhibit the splicing of the viral M1 mRNAs (**Fig. S6A**), in agreement with the previous findings that M1 mRNA splicing is little affected by RED or SMU1 silencing compared to NS1 mRNA splicing (23). Likewise, PB2 mRNA splicing, whose role in viral infection remains unclear (35), is not affected by 60 µM LSP641 or LSP61 (**Fig. S6B**). The specificity of LSP641 and LSP61 towards the splicing of NS1 mRNA, taken together with the findings that both compounds inhibit RED-SMU1 interaction (**Fig. 5A**) and IAV replication (**Fig. 6A**), with the observed effects being consistently more pronounced for LSP61, strongly argue for their antiviral effect being due to disruption of the RED-SMU1 complex.

### Example 2: Comparison of compounds LSP641, LSP61 and PD173074

As shown in **Figure 7****,** compounds LSP641 and LSP61 are structurally related to compound PD173074, which is a potent inhibitor of FGFR1 and FGFR3 (reported IC₅₀ 21.5 and 5 nM, respectively) (Mohammadi et al, 1998 - PMID 9774334) and thus poorly-selective (multi-kinase inhibitory activity).

The inventors compared compounds LSP641 and LSP61 to PD173074 in terms of (i) anti-FGFR3 activity, (ii) anti-viral activity as assessed using the WSN-PB2-Nanoluc reporter virus and (iii) RED-SMU1 disruption activity as assessed using the cell-based split-luciferase complementation assay.

The results are in **Figure 7****:** although the anti-FGFR3 activity of LSP61 and PD173074 were in the same range, PD173074 did not inhibit viral growth as efficiently as LSP61, suggesting that the anti-viral activity of LSP61 is related to its RED-SMU1 disrupting activity rather than its anti-FGFR3 activity.

Thus, contrary to PD173074, the compounds of formula (I) according to the invention are specific for RED-SMU1 disruption activity.

### References

The references of the present application are the following:
1. Kaufmann SHE, Dorhoi A, Hotchkiss RS, & Bartenschlager R (2018) Host-directed therapies for bacterial and viral infections. Nat Rev Drug Discov 17(1):35-56.
2. Zumla A, et al. (2016) Host-directed therapies for infectious diseases: current status, recent progress, and future prospects. Lancet Infect Dis 16(4):e47-63.
3. Krammer F, et al. (2018) Influenza. Nat Rev Dis Primers 4(1):3.
4. Hurt AC (2014) The epidemiology and spread of drug resistant human influenza viruses. Curr Opin Virol 8:22-29.
5. Lackenby A, et al. (2018) Global update on the susceptibility of human influenza viruses to neuraminidase inhibitors and status of novel antivirals, 2016-2017. Antiviral Res 157:38-46.
6. Hurt AC, et al. (2012) Characteristics of a widespread community cluster of H275Y oseltamivir-resistant A(H1 N1)pdm09 influenza in Australia. J Infect Dis 206(2):148-157.
7. Takashita E, et al. (2015) Characterization of a large cluster of influenza A(H1N1)pdm09 viruses cross-resistant to oseltamivir and peramivir during the 2013-2014 influenza season in Japan. Antimicrob Agents Chemother 59(5):2607-2617.
8. Meijer A, et al. (2009) Oseltamivir-resistant influenza virus A (H1N1), Europe, 2007-08 season. Emerg Infect Dis 15(4):552-560.
9. Bank C, et al. (2016) An experimental evaluation of drug-induced mutational meltdown as an antiviral treatment strategy. Evolution 70(11):2470-2484.
10. Goldhill DH, et al. (2018) The mechanism of resistance to favipiravir in influenza. Proc Natl Acad Sci U S A 115(45):11613-11618.
11. Hayden FG, et al. (2018) Baloxavir Marboxil for Uncomplicated Influenza in Adults and Adolescents. N Engl J Med 379(10):913-923.
12. Koszalka P, Tilmanis D, & Hurt AC (2017) Influenza antivirals currently in late-phase clinical trial. Influenza Other Respir Viruses 11(3):240-246.
13. McKimm-Breschkin JL, et al. (2018) Prevention and treatment of respiratory viral infections: Presentations on antivirals, traditional therapies and host-directed interventions at the 5th ISIRV Antiviral Group conference. Antiviral Res 149:118-142.
14. Ramos EL, et al. (2015) Efficacy and safety of treatment with an anti-m2e monoclonal antibody in experimental human influenza. J Infect Dis 211(7):1038-1044.
15. Wollacott AM, et al. (2016) Safety and Upper Respiratory Pharmacokinetics of the Hemagglutinin Stalk-Binding Antibody VIS410 Support Treatment and Prophylaxis Based on Population Modeling of Seasonal Influenza A Outbreaks. EBioMedicine 5:147-155.
16. Byrn RA, et al. (2015) Preclinical activity of VX-787, a first-in-class, orally bioavailable inhibitor of the influenza virus polymerase PB2 subunit. Antimicrob Agents Chemother 59(3):1569-1582.
17. Rossignol JF (2014) Nitazoxanide: a first-in-class broad-spectrum antiviral agent. Antiviral Res 110:94-103.
18. Haffizulla J, et al. (2014) Effect of nitazoxanide in adults and adolescents with acute uncomplicated influenza: a double-blind, randomised, placebo-controlled, phase 2b/3 trial. Lancet Infect Dis 14(7):609-618.
19. Colombo RE, et al. (2016) A phase 1 randomized, double-blind, placebo-controlled, crossover trial of DAS181 (Fludase(R)) in adult subjects with well-controlled asthma. BMC Infect Dis 16:54.
20. Zenilman JM, et al. (2015) Phase 1 clinical trials of DAS181, an inhaled sialidase, in healthy adults. Antiviral Res 123:114-119.
21. van de Wakker SI, Fischer MJE, & Oosting RS (2017) New drug-strategies to tackle viral-host interactions for the treatment of influenza virus infections. Eur J Pharmacol 809:178-190.
22. Watanabe T & Kawaoka Y (2015) Influenza virus-host interactomes as a basis for antiviral drug development. Curr Opin Virol 14:71-78.
23. Fournier G, et al. (2014) Recruitment of RED-SMU1 complex by Influenza A Virus RNA polymerase to control Viral mRNA splicing. PLoS Pathog 10(6):e1004164.
24. Bessonov S, Anokhina M, Will CL, Urlaub H, & Luhrmann R (2008) Isolation of an active step I spliceosome and composition of its RNP core. Nature 452(7189):846-850.
25. Chung T, Wang D, Kim CS, Yadegari R, & Larkins BA (2009) Plant SMU-1 and SMU-2 homologues regulate pre-mRNA splicing and multiple aspects of development. Plant Physiol 151(3):1498-1512.
26. Papasaikas P, Tejedor JR, Vigevani L, & Valcarcel J (2015) Functional splicing network reveals extensive regulatory potential of the core spliceosomal machinery. Mol Cell 57(1):7-22.
27. Spartz AK, Herman RK, & Shaw JE (2004) SMU-2 and SMU-1, Caenorhabditis elegans homologs of mammalian spliceosome-associated proteins RED and fSAP57, work together to affect splice site choice. Mol Cell Biol 24(15):6811-6823.
28. Hegele A, et al. (2012) Dynamic protein-protein interaction wiring of the human spliceosome. Mol Cell 45(4):567-580.
29. Ulrich AKC, Schulz JF, Kamprad A, Schutze T, & Wahl MC (2016) Structural Basis for the Functional Coupling of the Alternative Splicing Factors Smu1 and RED. Structure 24(5):762-773.
30. Gerard FC, et al. (2009) Modular organization of rabies virus phosphoprotein. J Mol Biol 388(5) :978-996.
31. Boxem M, et al. (2008) A protein domain-based interactome network for C. elegans early embryogenesis. Cell 134(3):534-545.
32. Le Corre L, et al. (2010) Synthesis and biological evaluation of a triazole-based library of pyrido[2,3-d]pyrimidines as FGFR3 tyrosine kinase inhibitors. Org Biomol Chem 8(9):2164-2173.
33. Matrosovich M, Matrosovich T, Carr J, Roberts NA, & Klenk HD (2003) Overexpression of the alpha-2,6-sialyltransferase in MDCK cells increases influenza virus sensitivity to neuraminidase inhibitors. J Virol 77(15):8418-8425.
34. Seitz C, Frensing T, Hoper D, Kochs G, & Reichl U (2010) High yields of influenza A virus in Madin-Darby canine kidney cells are promoted by an insufficient interferon-induced antiviral state. J Gen Virol 91 (Pt 7):1754-1763.
35. Yamayoshi S, Watanabe M, Goto H, & Kawaoka Y (2016) Identification of a Novel Viral Protein Expressed from the PB2 Segment of Influenza A Virus. J Virol 90(1):444-456.
36. Bertram K, et al. (2017) Cryo-EM Structure of a Pre-catalytic Human Spliceosome Primed for Activation. Cell 170(4):701-713 e711.
37. Arkin MR, Tang Y, & Wells JA (2014) Small-molecule inhibitors of protein-protein interactions: progressing toward the reality. Chem Biol 21(9):1102-1114.
38. Scott DE, Bayly AR, Abell C, & Skidmore J (2016) Small molecules, big targets: drug discovery faces the protein-protein interaction challenge. Nat Rev Drug Discov 15(8):533-550.
39. Zhang B, Golding BT, & Hardcastle IR (2015) Small-molecule MDM2-p53 inhibitors: recent advances. Future Med Chem 7(5):631-645.
40. Weydert C, De Rijck J, Christ F, & Debyser Z (2016) Targeting Virus-host Interactions of HIV Replication. Curr Top Med Chem 16(10):1167-1190.
41. Fosgerau K & Hoffmann T (2015) Peptide therapeutics: current status and future directions. Drug Discov Today 20(1):122-128.
42. Dassah M, Patzek S, Hunt VM, Medina PE, & Zahler AM (2009) A genetic screen for suppressors of a mutated 5' splice site identifies factors associated with later steps of spliceosome assembly. Genetics 182(3):725-734.
43. Kanno T, et al. (2017) A Genetic Screen for Pre-mRNA Splicing Mutants of Arabidopsis thaliana Identifies Putative U1 snRNP Components RBM25 and PRP39a. Genetics 207(4):1347-1359.
44. Verbist B, et al. (2015) Using transcriptomics to guide lead optimization in drug discovery projects: Lessons learned from the QSTAR project. Drug Discov Today 20(5):505-513.
45. Yeh PC, Yeh CC, Chen YC, & Juang YL (2012) RED, a spindle pole-associated protein, is required for kinetochore localization of MAD1, mitotic progression, and activation of the spindle assembly checkpoint. The Journal of biological chemistry 287(15):11704-11716.
46. Ren L, et al. (2013) Loss of Smu1 function de-represses DNA replication and over-activates ATR-dependent replication checkpoint. Biochem Biophys Res Commun 436(2):192-198.
47. Shah VJ & Maddika S (2018) CRL7(SMU1) E3 ligase complex-driven H2B ubiquitylation functions in sister chromatid cohesion by regulating SMC1 expression. J Cell Sci 131(8).
48. Jiang W, et al. (2013) Influenza A virus NS1 induces G0/G1 cell cycle arrest by inhibiting the expression and activity of RhoA protein. J Virol 87(6):3039-3052.
49. Fan Y, et al. (2017) Cell Cycle-independent Role of Cyclin D3 in Host Restriction of Influenza Virus Infection. J Biol Chem 292(12):5070-5088.
50. Teixeira VH, et al. (2013) Stochastic homeostasis in human airway epithelium is achieved by neutral competition of basal cell progenitors. Elife 2:e00966.
51. Nikolaidis NM, et al. (2017) Mitogenic stimulation accelerates influenza-induced mortality by increasing susceptibility of alveolar type II cells to infection. Proc Natl Acad Sci U S A 114(32):E6613-E6622.
52. Kaida D, et al. (2007) Spliceostatin A targets SF3b and inhibits both splicing and nuclear retention of pre-mRNA. Nat Chem Biol 3(9):576-583.
53. Kotake Y, et al. (2007) Splicing factor SF3b as a target of the antitumor natural product pladienolide. Nat Chem Biol 3(9):570-575.
54. Fan L, Lagisetti C, Edwards CC, Webb TR, & Potter PM (2011) Sudemycins, novel small molecule analogues of FR901464, induce alternative gene splicing. ACS Chem Biol 6(6):582-589.
55. Bonnal S, Vigevani L, & Valcarcel J (2012) The spliceosome as a target of novel antitumour drugs. Nat Rev Drug Discov 11(11):847-859.
56. Effenberger KA, Urabe VK, & Jurica MS (2017) Modulating splicing with small molecular inhibitors of the spliceosome. Wiley Interdiscip Rev RNA 8(2).
57. Hsu TY, et al. (2015) The spliceosome is a therapeutic vulnerability in MYC-driven cancer. Nature 525(7569):384-388.
58. Lagisetti C, et al. (2009) Synthetic mRNA splicing modulator compounds with in vivo antitumor activity. J Med Chem 52(22):6979-6990.
59. Lee SC, et al. (2016) Modulation of splicing catalysis for therapeutic targeting of leukemia with mutations in genes encoding spliceosomal proteins. Nat Med 22(6):672-678.
60. Jager S, et al. (2012) Global landscape of HIV-human protein complexes. Nature 481 (7381):365-370.
61. Konig R, et al. (2008) Global analysis of host-pathogen interactions that regulate early-stage HIV-1 replication. Cell 135(1):49-60.
62. Rozenblatt-Rosen O, et al. (2012) Interpreting cancer genomes using systematic host network perturbations by tumour virus proteins. Nature 487(7408):491-495.
63. Lee SC & Abdel-Wahab O (2016) Therapeutic targeting of splicing in cancer. Nat Med 22(9):976-986.
64. Salton M & Misteli T (2016) Small Molecule Modulators of Pre-mRNA Splicing in Cancer Therapy. Trends Mol Med 22(1):28-37.
65. Cassonnet P, et al. (2011) Benchmarking a luciferase complementation assay for detecting protein complexes. Nat Methods 8(12):990-992.
66. Kabsch W (2010) Integration, scaling, space-group assignment and post-refinement. Acta Crystallogr D Biol Crystallogr 66(Pt 2): 133-144.
67. de Sanctis D, et al. (2012) ID29: a high-intensity highly automated ESRF beamline for macromolecular crystallography experiments exploiting anomalous scattering. J Synchrotron Radiat 19(Pt 3):455-461.
68. Sheldrick GM (2010) Experimental phasing with SHELXC/D/E: combining chain tracing with density modification. Acta Crystallogr D Biol Crystallogr 66(Pt 4):479-485.
69. de La Fortelle E & Bricogne G (1997) [27] Maximum-likelihood heavy-atom parameter refinement for multiple isomorphous replacement and multiwavelength anomalous diffraction methods. Methods Enzymol 276:472-494.
70. Cowtan K (2006) The Buccaneer software for automated model building. 1. Tracing protein chains. Acta Crystallogr D Biol Crystallogr 62(Pt 9):1002-1011.
71. Cohen SX, et al. (2008) ARP/wARP and molecular replacement: the next generation. Acta Crystallogr D Biol Crystallogr 64(Pt 1):49-60.
72. Emsley P & Cowtan K (2004) Coot: model-building tools for molecular graphics. Acta Crystallogr D Biol Crystallogr 60(Pt 12 Pt 1):2126-2132.
73. McCoy AJ, et al. (2007) Phaser crystallographic software. J Appl Crystallogr 40(Pt 4):658-674.
74. Murshudov GN, Vagin AA, & Dodson EJ (1997) Refinement of macromolecular structures by the maximum-likelihood method. Acta Crystallogr D Biol Crystallogr 53(Pt 3):240-255.
75. Winn MD, et al. (2011) Overview of the CCP4 suite and current developments. Acta Crystallogr D Biol Crystallogr 67(Pt 4):235-242.
76. Diller DJ & Merz KM, Jr. (2001) High throughput docking for library design and library prioritization. Proteins 43(2):113-124.
77. The PyMOL Molecular Graphics System Version 2.0 Schrödinger LLC.

## Claims

1. Compound chosen from the compounds of formula (A), their enantiomers and their pharmaceutically acceptable salts: 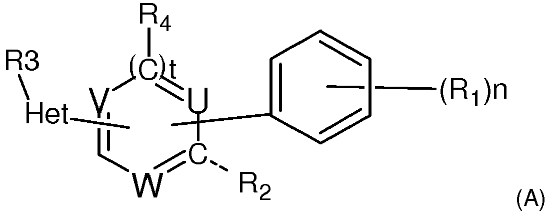 wherein:
U, V and W are each independently an atom chosen from C, N, O and S,
R1 is a radical chosen from C1-C6-alkoxy, -NO2, -NH2, -COOH, halogen and -O-CH2-CH2-COOH,
n is an integer comprised between 1 and 3,
R2 may be present or absent, and when present, is a radical chosen from -NH-CO-NH-Alk, -NH-CS-NH-Alk and -CH2-CO-NH-Alk, wherein Alk is a C1-C6 alkyl,
Het is a heteroaryle which may be fused to the aromatic cycle comprising U, V and W, and which may be substituted,
R3 is a radical chosen from -phenyl-triazole-aralkyle, -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
t is equal to 0 or 1, and R4 is a radical chosen from H, halogen and -C(R5)(R6)-CH2OH, wherein R5 and R6 each represent H or a C1-C6 alkyl,
for use for preventing and/or treating a viral infection.

2. Compound for use according to claim 1, wherein it chosen from the compounds of formula (A'), their enantiomers and their pharmaceutically acceptable salts: 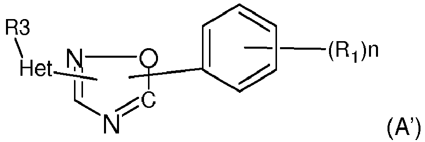
wherein R1, R3, Het and n are as in claim 1,
preferably Het is a heteroaryle which is not fused to the aromatic cycle comprising U, V and W, preferably a pyrazole, and may be substituted, preferably by at least one group - NH-CO-Alk, wherein Alk is a C1-C6 alkyl, and/or by at least one group -COO-Alk, wherein Alk is a C1-C6 alkyl,
preferably R3 is -phenyl-triazole-aralkyle, preferably the aralkyle is benzyl,
preferably, the compound of formula (A') is compound LSP958 as follows:

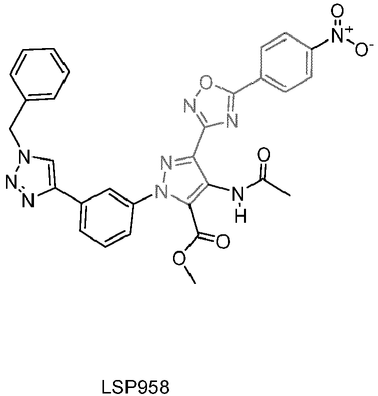

3. Compound for use according to claim 1, wherein it chosen from the compounds of formula (I), their enantiomers and their pharmaceutically acceptable salts: 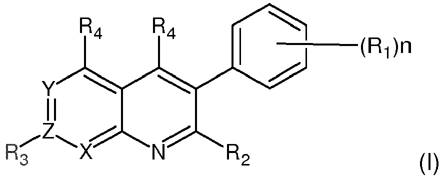 wherein:
X, Y and Z are each independently an atom chosen from C, N, O and S,
R1 is a radical chosen from C1-C6-alkoxy, -NH2, -COOH, halogen and -O-CH2-CH2-COOH,
n is an integer comprised between 1 and 3,
R2 is a radical chosen from -NH-CO-NH-Alk, -NH-CS-NH-Alk and -CH2-CO-NH-Alk, wherein Alk is a C1-C6 alkyl,
R3 is a radical chosen from -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
R4 is a radical chosen from H, halogen and -C(R5)(R6)-CH2OH, wherein R5 and R6 each represent H or a C1-C6 alkyl.

4. Compound for use according to any one of claims 1 to 3, wherein the viral infection is an infection by a virus chosen from influenza viruses, human immunodeficiency viruses and human papillomaviruses, preferably chosen from Influenza virus A, Influenza virus B, Influenza virus C, Influenza virus D, HIV-1, HIV-2, HPV16, HPV18, HPV6 and HPV11.

5. Compound for use according to any one of claims 1 to 4, wherein the viral infection is an infection by Influenza virus A.

6. Compound chosen from the compounds of formula (A), their enantiomers and their pharmaceutically acceptable salts: 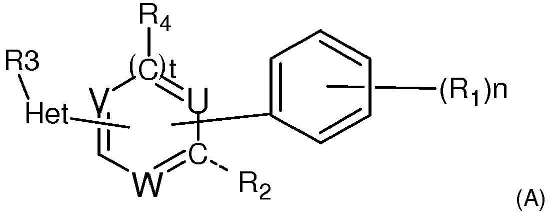 wherein:
U, V and W are each independently an atom chosen from C, N, O and S,
R1 is a radical chosen from C1-C6-alkoxy, -NO2, -NH2, -COOH, halogen and -O-CH2-CH2-COOH,
n is an integer comprised between 1 and 3,
R2 may be present or absent, and when present, is a radical chosen from -NH-CO-NH-Alk, -NH-CS-NH-Alk and -CH2-CO-NH-Alk, wherein Alk is a C1-C6 alkyl,
Het is a heteroaryle which may be fused to the aromatic cycle comprising U, V and W, and which may be substituted,
R3 is a radical chosen from -phenyl-triazole-aralkyle, -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
t is equal to 0 or 1, and R4 is a radical chosen from H, halogen and -C(R5)(R6)-CH2OH, wherein R5 and R6 each represent H or a C1-C6 alkyl,
for use for preventing and/or treating cancer.

7. Compound for use according to claim 6, wherein it chosen from the compounds of formula (I), their enantiomers and their pharmaceutically acceptable salts: 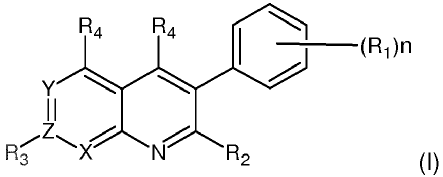 wherein:
X, Y and Z are each independently an atom chosen from C, N, O and S,
R1 is a radical chosen from C1-C6-alkoxy, -NH2, -COOH, halogen and -O-CH2-CH2-COOH,
n is an integer comprised between 1 and 3,
R2 is a radical chosen from -NH-CO-NH-Alk, -NH-CS-NH-Alk and -CH2-CO-NH-Alk, wherein Alk is a C1-C6 alkyl,
R3 is a radical chosen from -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
R4 is a radical chosen from H, halogen and -C(R5)(R6)-CH2OH, wherein R5 and R6 each represent H or a C1-C6 alkyl.

8. Compound for use according to claim 6 or 7, wherein the cancer is a colon cancer, a colorectal cancer, a melanoma, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a renal cancer, a skin cancer, a stomach cancer, a testis cancer, an urothelial cancer, an adrenocortical carcinoma or a non solid cancer such as lymphoma.

9. Compound for use according to any one of claims 1 to 8, wherein it disrupts and/or destabilizes the RED-SMU1 complex.

10. Compound for use according to any one of claims 1 to 9, wherein the fluorophore is selected from fluorescein-type fluorophores, rhodamine-type fluorophores, xanthine-type fluorophores, naphthalene-type fluorophores such as dansyl-type fluorophores, carbocyanine-type fluorophores, coumarin-type fluorophores, acridine-type fluorophores, pyrene-type fluorophores and anthracene-type fluorophores, preferably the fluorophore is a fluorescein-type fluorophore, more preferably fluorescein.

11. Compound for use according to any one of claims 1 to 10, wherein the heterocycle is a monocyclic or polycyclic saturated hydrocarbon group in which at least one carbon atom of the ring is substituted by a heteroatom, and which may be optionally substituted, preferably the heterocycle is morpholino, tetrahydropyrane, oxetane or azetidine, more preferably the heterocycle is 1-benzyl-4-morpholino.

12. Compound for use according to any one of claims 3 to 5 or 7 to 11, wherein it comprises at least one of the following features:
- Y and X are each N; and/or
- Z is C; and/or
- n is 2, and preferably both R1 are in ortho positions; and/or
- R1 is a C1-C6 alkoxy, more preferably methoxy; and/or
- R2 is the radical -NH-CO-NH-Alk, wherein Alk is a C1-C6 alkyl; and/or
- R3 is a radical chosen from -NH-(CH2)m-alkyne, -NH-(CH2)m-triazole-fluorophore and - NH-(CH2)m-triazole-heterocycle, wherein m is 1, 2 or 3; and/or
- R4 is H.

13. Compound for use according to any one of claims 1 to 12, wherein it is chosen from:

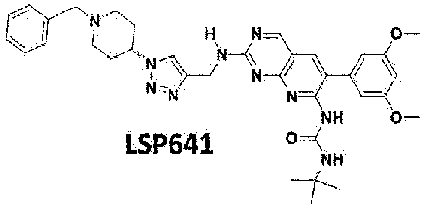

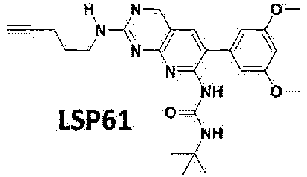

14. Compound chosen from the compounds of formula (II), their enantiomers and their pharmaceutically acceptable salts: 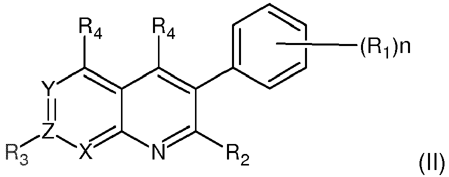 wherein:
X, Y and Z are each independently an atom chosen from C, N, O and S,
R1 is a radical chosen from C1-C6-alkoxy, -NH2, -COOH, halogen and -O-CH2-CH2-COOH,
n is an integer comprised between 1 and 3,
R2 is a radical chosen from -NH-CO-NH-Alk, -NH-CS-NH-Alk and -CH2-CO-NH-Alk, wherein Alk is a C1-C6 alkyl,
R3 is a radical chosen from -NH-(CH2)m-alkyne, -NH-(CH2)m-OH, -NH-(CH2)m-triazole-fluorophore and -NH-(CH2)m-triazole-heterocycle, wherein m is an integer comprised between 1 and 5, and
R4 is a radical chosen from H, halogen and -C(R5)(R6)-CH2OH, wherein R5 and R6 each represent H or a C1-C6 alkyl, provided that either at least one R4 is different from H, or, when both R4 are H, then R3 is -NH-(CH2)m-triazole-heterocycle with the heterocycle being 1-benzyl-4-morpholino.

15. Compound according to claim 14, wherein it is:

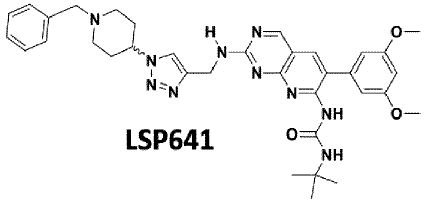

16. Compound according to claim 14 or 15, for use as a medicament.

17. Process for high-throughput pharmacological screening of compounds which disrupt and/or destabilize the RED-SMU1 complex, comprising:
a) mixing a test compound with a peptide comprising at least the sequence SEQ ID NO:3, and preferably mixing a test compound with the peptide consisting of SEQ ID NO:3 or SEQ ID NO:5;
b) adding a compound conjugated to a fluorophore according to any one of claims 1 to 13 to the mixture obtained in a); and
c) measuring the binding of the conjugated compound in the mixture obtained in step b),
wherein if the binding measured in step c) is less than the binding measured in a control, then concluding that the test compound disrupts and/or destabilizes the RED-SMU1 complex, and
preferably the control is a mixture comprising the same compound conjugated to a fluorophore as the one of step b), and the same peptide comprising at least the sequence SEQ ID NO:3 as the one of step a).
